# EUROPEAN PATENT SPECIFICATION

(11) **EP 4 213 921 B1**
(45) Date of publication and mention of the grant of the patent: **18.02.2026**
(21) Application number: 21867952.0
(22) Date of filing: 17.09.2021
(51) Int. Cl.: A61M 16/06, A61M 16/08

(54) **POSITIONING AND STABILISING STRUCTURE WITH TEXTILE SLEEVE**
POSITIONIER- UND STABILISIERUNGSSTRUKTUR MIT TEXTILHÜLLE
STRUCTURE DE POSITIONNEMENT ET DE STABILISATION À MANCHON TEXTILE

(30) Priority: 18.09.2020 AU 2020903344; 18.09.2020 AU 2020903345; 21.06.2021 SG 10202106712T
(43) Date of publication of application: 26.07.2023
(73) Proprietor: ResMed Asia Pte. Ltd., Singapore 639443 (SG)
(72) Inventor: KHERA, Rahul, Bella Vista, New South Wales 2153 (AU); SUPAOPASPHUN, Thontira, Singapore 639443 (SG); TAN, Bangzheng, Singapore 639443 (SG); THA, Nwe Oo, Bella Vista, New South Wales 2153 (AU); VALIYAMBATH, Mohankumar Krishnan, Singapore 639443 (SG)
(74) Representative: Vossius & Partner Patentanwälte Rechtsanwälte mbB
(86) International application number: PCT/AU2021/051076
(87) International publication number: WO 2022/056595

(56) References cited:
- WO-A1-2008/011683
- WO-A1-2017/124152
- US-A1- 2019 022 343
- US-A1- 2019 282 777
- US-A1- 2020 009 345

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 1.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO₂ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of at least 6 H₂O, e.g. about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface. a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 1.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

One form of positioning and stabilising structure comprises a pair of gas delivery tubes to receive the flow of air from a connection port on top of the patient's head and to deliver the flow of air to the entrance of the patient's airways via the seal-forming structure. In examples the gas delivery tubes may be made from silicone.

Textile sleeves may be provided over the gas delivery tubes to avoid contact between the tube surface and the patient's face. However, since such sleeves are typically opaque, the use of such sleeves may prevent the patient from visually confirming the cleanliness of the gas delivery tube. Patients may be uncomfortable using equipment if the cleanliness of that equipment, particularly of components in the air flow path, cannot be confirmed.

If textile sleeves are provided, it may be desirable to ensure that the sleeves are permanently connected to the gas delivery tubes, so as to ensure that the sleeve does not bunch up (e.g. gather or wrinkle) or roll up, or move away from its correct position on the tubes. However, the presence of the sleeves should not impact on the ability of the tubes to resiliently extend, and the sleeves, if provided, should be comfortable against the patient's skin.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH₂O).

| RPT Device name | A-weighted sound pressure level dB(A) | Year (approx.) |
|---|---|---|
| C-Series Tango^{™} | 31.9 | 2007 |
| C-Series Tango^{™} with Humidifier | 33.1 | 2007 |
| S8 Escape^{™} II | 30.5 | 2005 |
| S8 Escape^{™} II with H4i^{™} Humidifier | 31.1 | 2005 |
| S9 AutoSet^{™} | 26.5 | 2010 |
| S9 AutoSet^{™} with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar^{™} Series of Adult and Paediatric Ventilators may provide support for invasive and non-invasive non-dependent ventilation for a range of patients for treating a number of conditions such as but not limited to NMD, OHS and COPD.

The ResMed Elisée^{™} 150 ventilator and ResMed VS III^{™} ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 1.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

**Table of noise of prior masks (ISO 17510-2:2007, 10 cmH₂O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage^{™} (*) | nasal | 29.5 | 21.5 | 1998 |
| ResMed UltraMirage^{™} | nasal | 36 (3) | 28 (3) | 2000 |
| ResMed Mirage Activa^{™} | nasal | 32 (3) | 24 (3) | 2002 |
| ResMed Mirage Micro^{™} | nasal | 30 (3) | 22 (3) | 2008 |
| ResMed Mirage^{™} SoftGel | nasal | 29 (3) | 22 (3) | 2008 |
| ResMed Mirage^{™} FX | nasal | 26 (3) | 18 (3) | 2010 |
| ResMed Mirage Swift^{™} (*) | nasal pillows | 37 | 29 | 2004 |
| ResMed Mirage Swift^{™} II | nasal pillows | 28 (3) | 20 (3) | 2005 |
| ResMed Mirage Swift^{™} LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 2 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a patient interface comprising a plenum chamber pressurisable to a therapeutic pressure, a seal-forming structure constructed and arranged to form a seal with a region of the patient's face surrounding an entrance to the patient's airways for sealed delivery of a flow of air at the therapeutic pressure, and a positioning and stabilising structure to provide a force to hold a seal-forming structure in a therapeutically effective position on a patient's head.

One form of the present technology comprises a patient interface comprising:
a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient;
a seal-forming structure constructed and arranged to form a seal with a region of the patient's face surrounding an entrance to the patient's airways for sealed delivery of a flow of air at a therapeutic pressure of at least 6 cmH₂O above ambient air pressure throughout the patient's respiratory cycle in use, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; and
a positioning and stabilising structure to provide a force to hold a seal-forming structure in a therapeutically effective position on a patient's head, the seal-forming structure constructed and arranged to form a seal with a region of the patient's face surrounding an entrance to the patient's airways for sealed delivery of a flow of air at a therapeutic pressure of at least 6 cmH₂O above ambient air pressure to at least the patient's nares throughout the patient's respiratory cycle in use.

One form of the present technology comprises a positioning and stabilising structure to provide a force to hold a seal-forming structure in a therapeutically effective position on a patient's head.

One form of the present technology comprises an elongate textile sleeve configured to be provided around a gas delivery tube and arranged to be in contact, in use, with the patient's face, the sleeve comprising a wall with an opening therein for allowing the patient to view a portion of the gas delivery tube when the positioning and stabilising structure is not in use.

One form of the present technology comprises a positioning and stabilising structure to provide a force to hold a seal-forming structure in a therapeutically effective position on a patient's head, the seal-forming structure constructed and arranged to form a seal with a region of the patient's face surrounding an entrance to the patient's airways for sealed delivery of a flow of air at a therapeutic pressure of at least 6 cmH₂O above ambient air pressure to at least the patient's nares throughout the patient's respiratory cycle in use, the positioning and stabilising structure comprising:
a gas delivery tube to receive the flow of air from a connection port on top of the patient's head and to deliver the flow of air to the entrance of the patient's airways via the seal-forming structure, the gas delivery tube being constructed and arranged to contact, in use, at least a region of the patient's head superior to an otobasion superior of the patient's head,
the positioning and stabilising structure further comprising an elongate textile sleeve provided around the gas delivery tube and arranged to be in contact, in use, with the patient's face, the sleeve comprising a wall with an opening therein for allowing the patient to view a portion of the gas delivery tube when the positioning and stabilising structure is not in use.

In examples
a) the textile sleeve is resiliently flexible in an axial direction; b) the textile sleeve is resiliently flexible in a circumferential direction; c)the textile sleeve has a greater flexibility in the axial direction than the circumferential direction; d) the opening is substantially oval, elliptical or stadium shaped; e) the gas delivery tube comprises a concertina portion and a non-concertina portion, wherein the non-concertina portion is on the opposite side of the concertina portion to the connection port, wherein the textile sleeve is arranged such that the entire edge of the opening lies over the non-concertina portion of the gas delivery tube; f) the sleeve extends to the end of the non-concertina portion of the gas delivery tube which is opposite the concertina portion; g) the gas delivery tube comprises a strap engaging portion for engaging a strap, and the textile sleeve is arranged such that the strap engaging portion protrudes through the opening; h) the strap engaging portion comprises a tab; i) there is a clearance between the edge of the opening and the strap engaging portion; j) the clearance is in the longitudinal direction; k) the clearance allows the gas delivery tube to stretch, in use, without the strap engaging portion contacting the edge of the opening; 1) the opening is substantially 65mm long; m) the opening is less than 65mm long; n) there is a lateral clearance between the tab and each lateral edge of the opening of less than 5mm; o) the width of the opening is less than 50% of the circumference of the gas delivery tube, when the opening and the tube are measured at the same longitudinal position; p) the positioning and stabilising structure comprises a second strap engaging portion and the textile sleeve comprises a second said opening, wherein the textile sleeve is arranged such that the second strap engaging portion protrudes through the second opening; q) the positioning and stabilising structure comprises a second gas delivery tube and the textile sleeve extends over both gas delivery tubes; r) the textile sleeve comprises a first material on a patient contacting side of the sleeve, and a second material on a non patient contacting side of the sleeve; s) the first material is connected to the second material by a seam, wherein the seam is configured to allow stretch in the circumferential direction; t) the first material comprises a moisture wicking knit material; u) the second material comprises a smooth surface; v) seam tape is provided to the edge of the opening to reduce or eliminate fraying of the textile; w) the textile sleeve comprises a connection port opening configured to allow an air circuit or an elbow to be connected to the connection port, in use: x) the textile sleeve comprises seam tape provided to the edge of the connection port opening; and/or y) the seam tape provided to the edge of the connection port opening is provided to an inner surface of the textile sleeve.

Another form of the present technology comprises a positioning and stabilising structure to provide a force to hold a seal-forming structure in a therapeutically effective position on a patient's head, the seal-forming structure constructed and arranged to form a seal with a region of the patient's face surrounding an entrance to the patient's airways for sealed delivery of a flow of air at a therapeutic pressure of at least 6 cmH₂O above ambient air pressure to at least the patient's nares throughout the patient's respiratory cycle in use, the positioning and stabilising structure comprising:
a gas delivery tube to receive the flow of air from a connection port on top of the patient's head and to deliver the flow of air to the entrance of the patient's airways via the seal-forming structure, the gas delivery tube being constructed and arranged to contact, in use, at least a region of the patient's head superior to an otobasion superior of the patient's head,
the positioning and stabilising structure further comprising an elongate textile sleeve provided around the gas delivery tube and arranged to be in contact, in use, with the patient's face, wherein the sleeve comprises a single-piece knitted and/or woven structure that is resiliently flexible in both a circumferential direction and an axial direction.

In examples:
a) the textile sleeve comprises a main structure and one or more functional zones knitted and/or woven into the main structure, each said functional zone having one or more textile properties that are different than those of the main structure; b) at least one of said functional zones is a transparent or translucent zone through which at least a portion of the gas delivery tube is visible; c) the one or more textile properties comprise one or more of: knitting density; weaving density; a number of loops in a knit pattern; a pattern of the knit or weave; yarn density; yarn type; and fibre cross-section; d) at least part of the textile sleeve is coated or impregnated with at least one material conferring functional and/or aesthetic properties; e) the at least one material is one or more of: a phosphorescent material, a luminescent material, or an anti-microbial composition; f) the textile sleeve has a greater flexibility in the axial direction than the circumferential direction; g) the gas delivery tube comprises a strap engaging portion, and the textile sleeve has a first opening through which at least part of the strap engaging portion is accessible for engaging a strap; h) the strap engaging portion comprises a tab; i) the positioning and stabilising structure comprises a second strap engaging portion and the textile sleeve comprises a second opening through which the second strap engaging portion is accessible for engaging a strap; j) the positioning and stabilising structure comprises a second gas delivery tube and the textile sleeve extends over both gas delivery tubes; k) the textile sleeve comprises a connection port opening configured to allow an air circuit or an elbow to be connected to the connection port, in use; 1) the first opening is substantially oval, elliptical or stadium shaped; and/or, if present, the second opening is substantially oval, elliptical or stadium shaped; m) at least one of the first opening, the second opening, and the connection port opening comprises an edge reinforcement structure to reduce or eliminate fraying of the textile; and/or n) the edge reinforcement structure comprises one or more of: seam tape; stitching; a heat bonded section; a thickened region; and an end cap.

Another form of the present technology comprises a textile sleeve for a positioning and stabilising structure for providing a force to hold a seal-forming structure in a therapeutically effective position on a patient's head, the positioning and stabilising structure comprising a gas delivery tube to receive the flow of air from a connection port on top of the patient's head and to deliver the flow of air to the entrance of the patient's airways via the seal-forming structure, the textile sleeve being arranged, in use, to fit around the gas delivery tube and contact with the patient's face, the textile sleeve comprising a single-piece knitted and/or woven structure that is resiliently flexible in both a circumferential direction and an axial direction.

In examples:
a) the textile sleeve comprises a main structure and one or more functional zones knitted and/or woven into the main structure, each said functional zone having one or more textile properties that are different than those of the main structure; b) at least one of said functional zones is a transparent or translucent zone through which at least a portion of the gas delivery tube is visible when the textile sleeve is fitted to the positioning and stabilising structure; c) the one or more textile properties comprise one or more of: knitting density; weaving density; a number of loops in a knit pattern; a pattern of the knit or weave; yarn density: yarn type; and fibre cross-section; d) at least part of the textile sleeve is coated or impregnated with at least one material conferring functional and/or aesthetic properties; e) the at least one material is one or more of: a phosphorescent material, a luminescent material, or an anti-microbial composition; f) the textile sleeve has a greater flexibility in the axial direction than the circumferential direction; g) the textile sleeve has a first opening through which at least part of a strap engaging portion of the gas delivery tube is accessible for engaging a strap when the textile sleeve is fitted to the gas delivery tube; h) the strap engaging portion comprises a tab; i) the textile sleeve comprises a second opening through which a second strap engaging portion of the gas delivery tube is accessible for engaging a strap when the textile sleeve is fitted to the gas delivery tube; j) the textile sleeve is adapted to extend over both the gas delivery tube and a second gas delivery tube of the positioning and stabilising structure; k) the textile sleeve comprises a connection port opening configured to allow an air circuit or an elbow to be connected to the connection port, in use: 1) the first opening is substantially oval, elliptical or stadium shaped; and/or, if present, the second opening is substantially oval, elliptical or stadium shaped; m) at least one of the first opening, the second opening, and the connection port opening comprises an edge reinforcement structure to reduce or eliminate fraying of the textile; and/or n) the edge reinforcement structure comprises one or more of: seam tape; stitching; a heat bonded section; a thickened region; and an end cap.

Another form of the present technology comprises a positioning and stabilising structure to provide a force to hold a seal-forming structure in a therapeutically effective position on a patient's head, the seal-forming structure constructed and arranged to form a seal with a region of the patient's face surrounding an entrance to the patient's airways for sealed delivery of a flow of air at a therapeutic pressure of at least 6 cmH₂O above ambient air pressure to at least the patient's nares throughout the patient's respiratory cycle in use, the positioning and stabilising structure comprising:
at least one gas delivery tube to receive the flow of air from a connection port on top of the patient's head and to deliver the flow of air to the entrance of the patient's airways via the seal-forming structure, the at least one gas delivery tube being constructed and arranged to contact, in use, at least a region of the patient's head superior to an otobasion superior of the patient's head,
at least one elongate textile sleeve provided around at least a portion of the at least one gas delivery tube and arranged to be in contact, in use, with the patient's face,
wherein the at least one textile sleeve is secured relative to the at least one gas delivery tube at a position proximal to an end of the at least one gas delivery tube proximal to the seal-forming structure.

In examples:
a) the textile sleeve is secured to the at least one gas delivery tube by an adhesive; b) a layer of seam tape is provided over an end of the textile sleeve adjacent the gas delivery tube; c) the seam tape is connected to the textile sleeve by an adhesive; d) the at least one textile sleeve is secured relative to the at least one gas delivery tube by an end cap; e) the end cap extends from an outer surface of the at least one textile sleeve, over an end of the at least one textile sleeve, and over at least a portion of a rim of the at least one gas delivery tube; f) the end cap comprises an annular side wall and an end flange extending radially inwardly from the side wall; g) the side wall comprises at least one adhesive recess; h) the at least one adhesive recess is annular; i) the at least one adhesive recess is offset from an end of the side wall distal from the end flange; j) the at least one adhesive recess is offset from the end flange; k) the side wall comprises at least one axial recess extending from an end of the side wall towards the end flange; 1) at least one seam of the textile sleeve is received in the at least one axial recess; m) the at least one seam of the textile sleeve comprises a first seam and a second seam, and the at least one axial recess comprises a first axial recess and a second axial recess, and the first seam is received in the first axial recess, and the second seam is received in the second axial recess; n) the end cap comprises at least one visual indicator indicating one or more of: alignment of the at least one gas delivery tube to a connection to the seal-forming structure, and size of the positioning and stabilising structure; o) the at least one textile sleeve is secured relative to the at least one gas delivery tube by a localised high friction interface; p) the localised high friction interface is provided by a polymer layer between the textile sleeve and the at least one gas delivery tube; q) the polymer layer is a polymer tape; r) the polymer tape is a thermoplastic polyurethane tape; s) the polymer tape is a silicone tape; t) the polymer layer is a silicone layer; u) the at least one textile sleeve is secured relative to the at least one gas delivery tube by a double-sided tape between the at least one textile sleeve and the at least one gas delivery tube; v) the double-sided tape is coated with a silicone adhesive on a first side, and a non-silicone adhesive on a second side; w) the non-silicone adhesive is an acrylic adhesive; x) the at least one textile sleeve is secured relative to the at least one gas delivery tube by an overmoulded end portion; y) the overmoulded end portion extends from an outer surface of the at least one textile sleeve, over an end of the at least one textile sleeve, and over at least a portion of a rim of the at least one gas delivery tube; z) the overmoulded end portion comprises at least one visual indicator indicating one or more of: alignment of the at least one gas delivery tube to a connection to the seal-forming structure, and size of the positioning and stabilising structure; aa) the at least one gas delivery tube is made of an elastomeric material; bb) the at least one gas delivery tube comprises a pair of gas delivery tubes and the textile sleeve extends over both gas delivery tubes; cc) the at least one textile sleeve is secured relative to both of the gas delivery tubes proximal to the respective ends proximal to the seal-forming structure; and/or dd) the at least one textile sleeve is also secured to the at least one gas delivery tube proximal to the connection port.

Another form of the present technology comprises a patient interface comprising:
a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient;
a seal-forming structure constructed and arranged to form a seal with a region of the patient's face surrounding an entrance to the patient's airways for sealed delivery of a flow of air at a therapeutic pressure of at least 6 cmH₂O above ambient air pressure throughout the patient's respiratory cycle in use, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; and
a positioning and stabilising structure according to one of the forms of the technology set out above. WO2017/124152 describes a positioning and stabilising structure with an adjustable headgear tubing for a patient interface.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:

### 3.1 RESPIRATORY THERAPY SYSTEMS

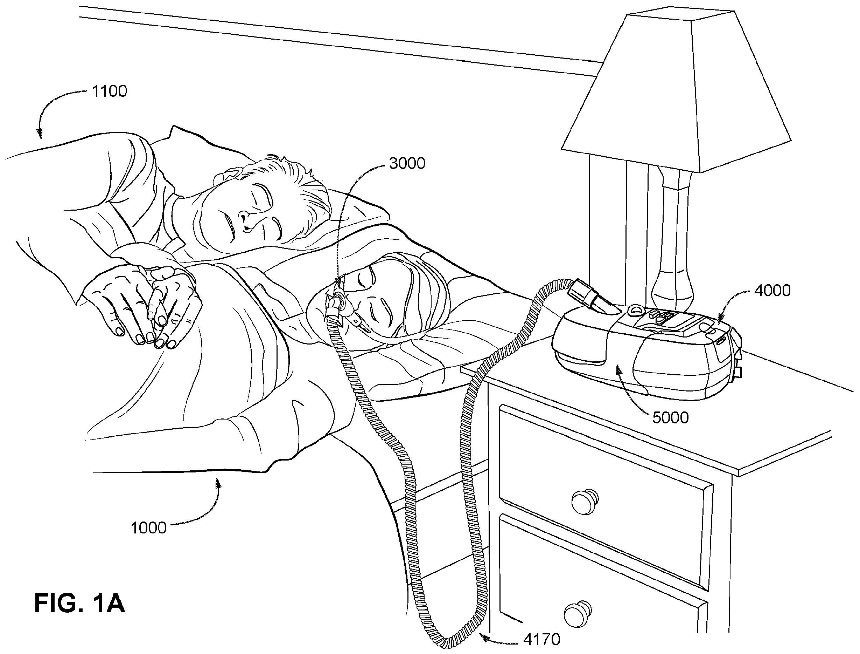

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

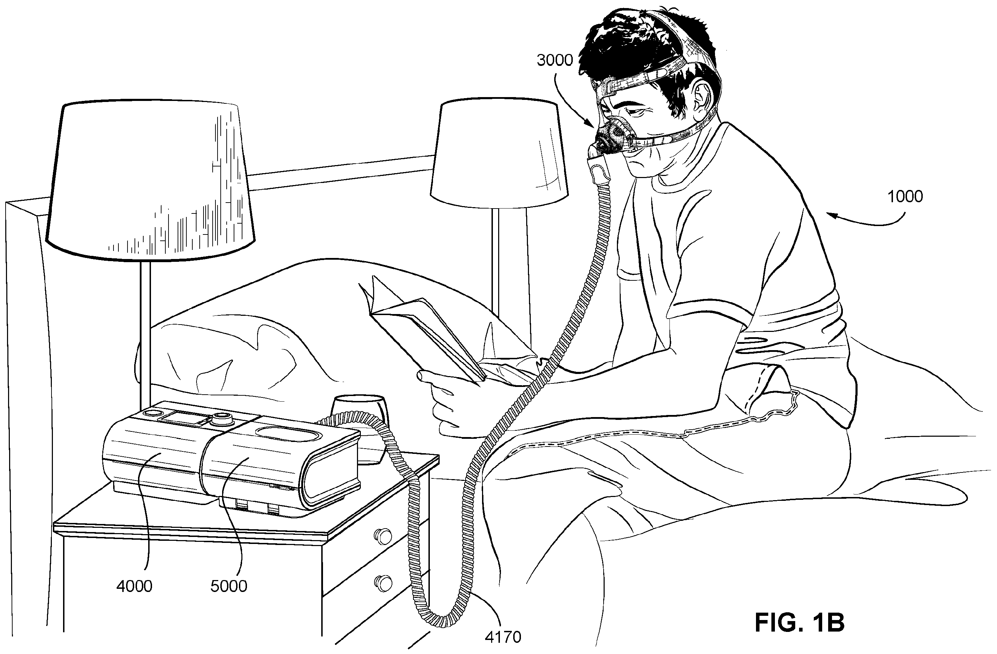

Fig. 1B shows a system including a patient 1000 wearing a patient interface 3000, in the form of a nasal mask. receiving a supply of air at positive pressure from an RPT device 4000. Air from the RPT device is humidified in a humidifier 5000, and passes along an air circuit 4170 to the patient 1000.

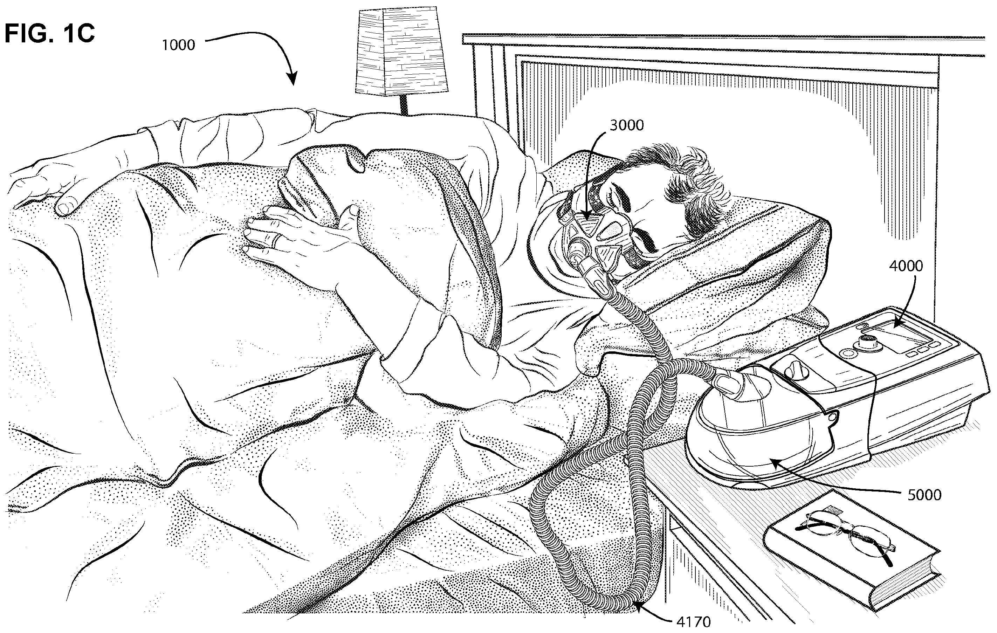

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 3.2 RESPIRATORY SYSTEM AND FACIAL ANATOMY

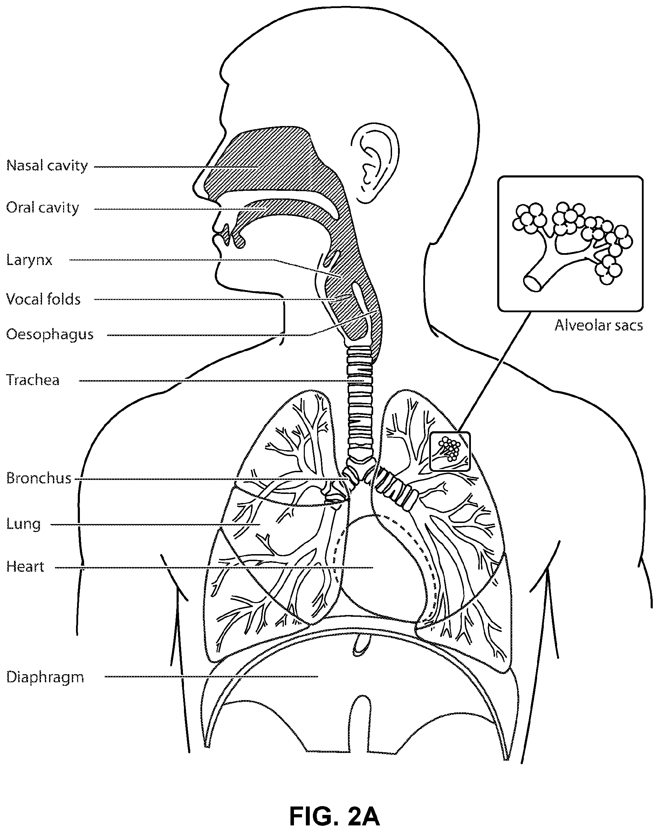

Fig. 2A shows an overview of a human respiratory system including the nasal and oral cavities, the larynx, vocal folds, oesophagus, trachea, bronchus, lung, alveolar sacs, heart and diaphragm.

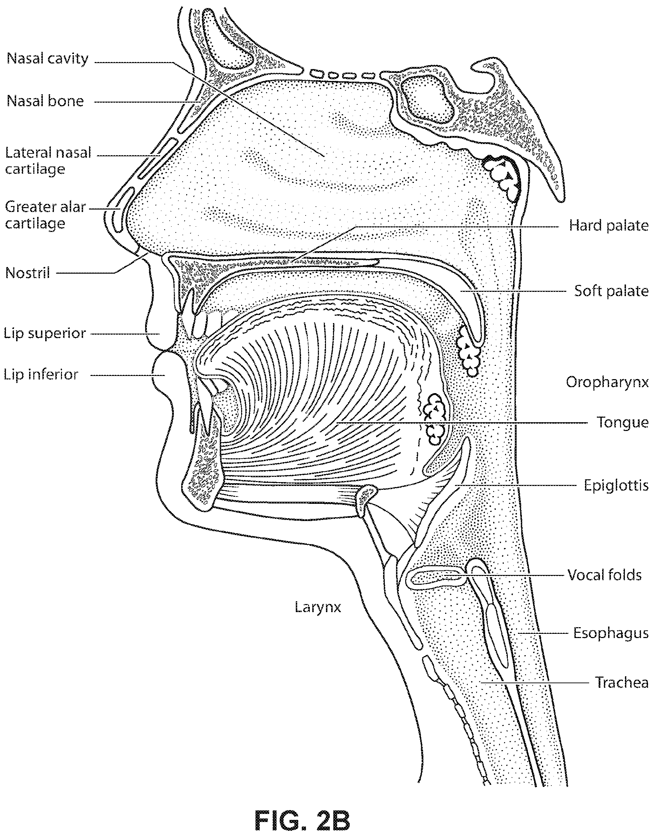

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

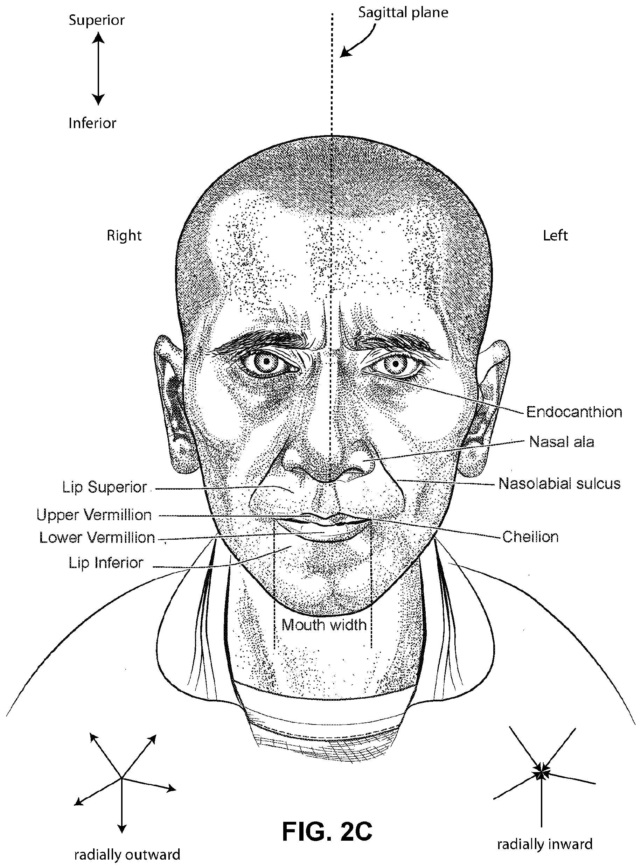

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

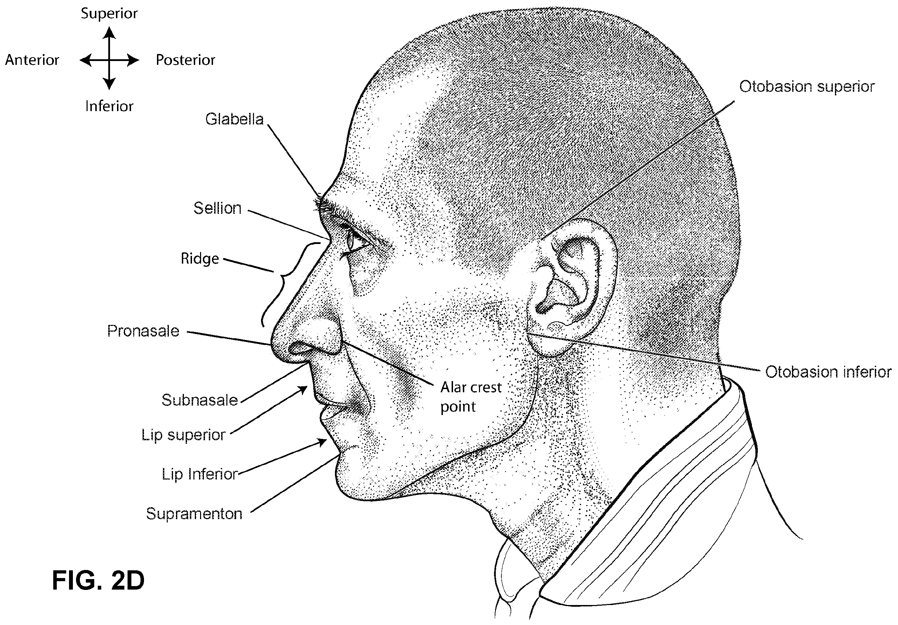

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

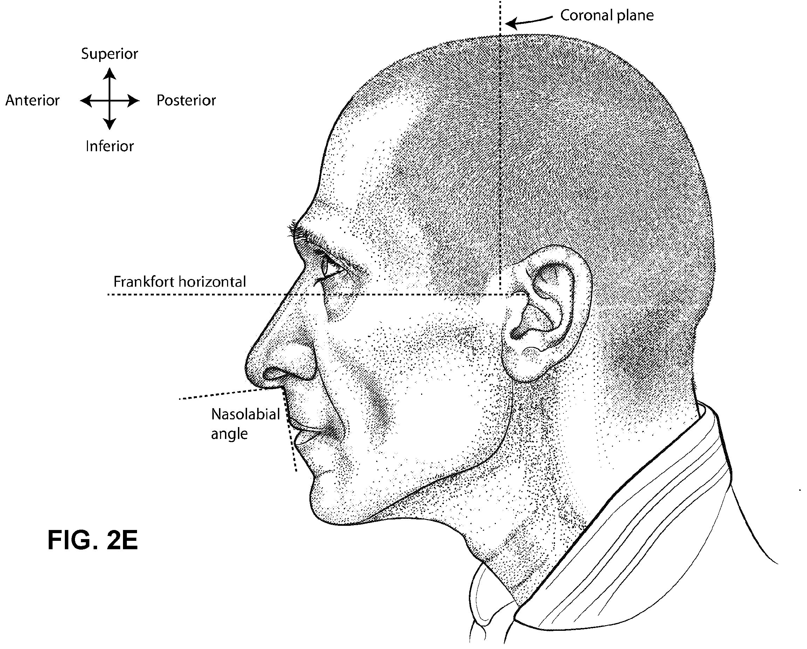

Fig. 2E is a further side view of a head. The approximate locations of the Frankfort horizontal and nasolabial angle are indicated. The coronal plane is also indicated.

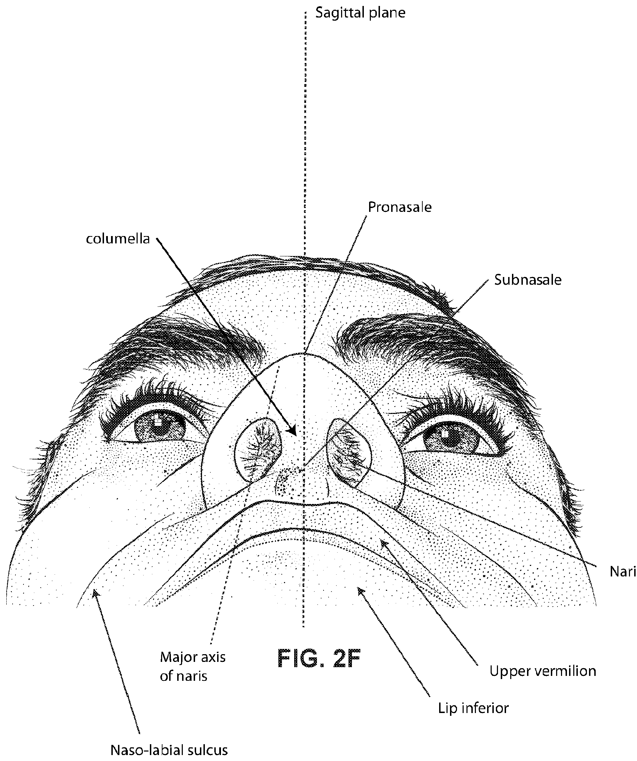

Fig. 2F shows a base view of a nose with several features identified including naso-labial sulcus, lip inferior, upper Vermilion, naris, subnasale, columella, pronasale, the major axis of a naris and the midsagittal plane.

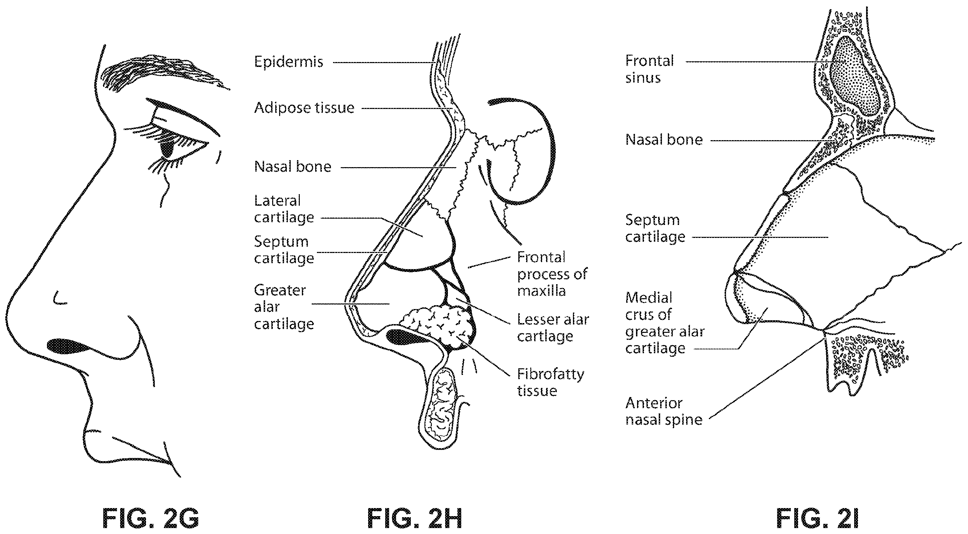

Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the midsagittal plane, amongst other things showing the septum cartilage and medial crus of greater alar cartilage.

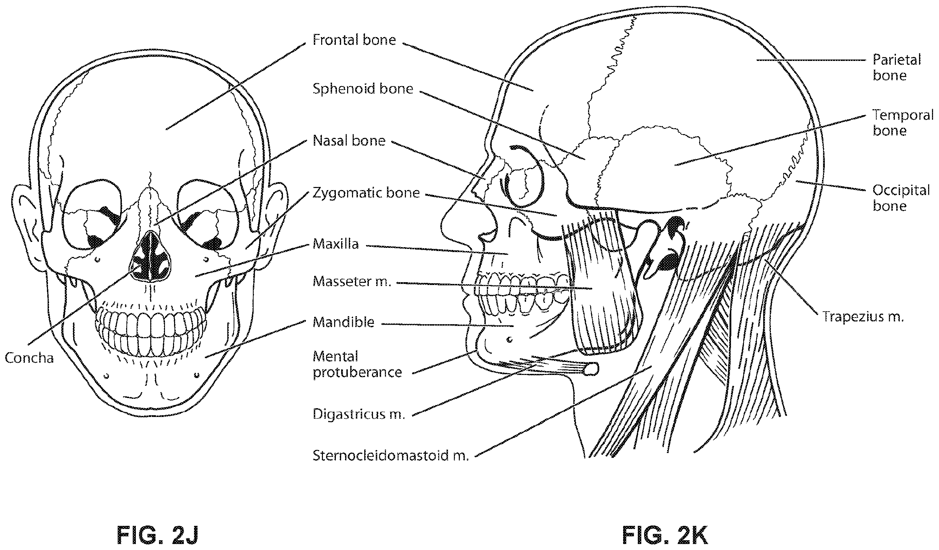

Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

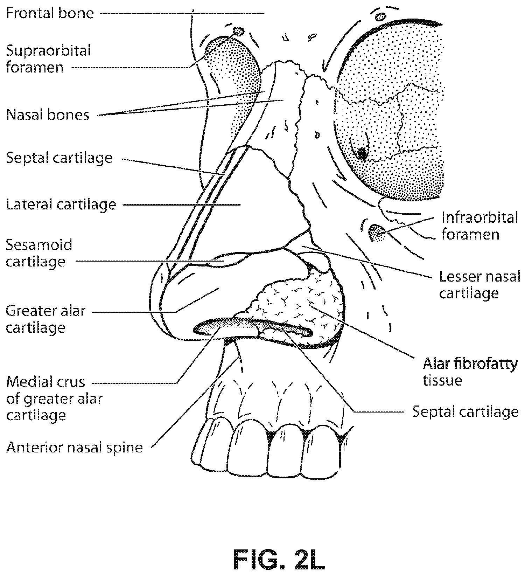

Fig. 2L shows an anterolateral view of a nose.

### 3.3 PATIENT INTERFACE

Fig. 3A shows a patient interface in the form of a nasal mask in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

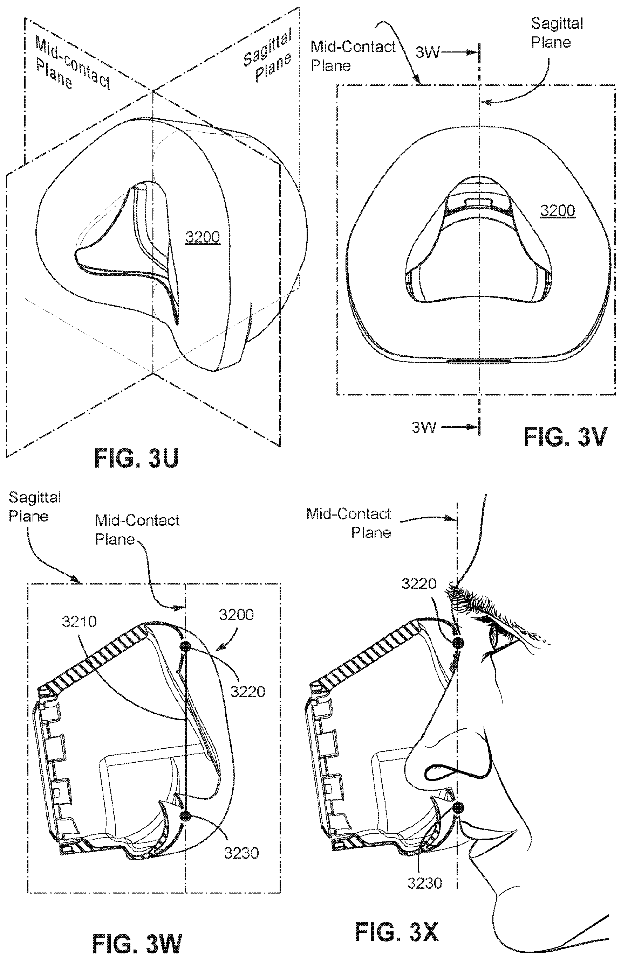

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.
Fig. 4 shows a perspective view of a patient interface in use.

### 3.4 PATIENT INTEFACES OF THE PRESENT TECHNOLOGY

Fig. 5 shows a perspective view of a patient interface comprising a textile sleeve according to one form of the technology, arranged in an in-use orientation.
Fig. 6 shows a top view of a positioning and stabilising structure comprising a textile sleeve according to one form of the technology.
Fig. 7 shows a bottom view of a positioning and stabilising structure comprising a textile sleeve according to one form of the technology.
Fig. 8 shows an enlarged top view of a gas delivery tube of a positioning and stabilising structure, with the edge of an opening of a textile sleeve according to one form of the invention shown, but the remainder of the textile sleeve omitted for clarity.
Fig. 9 shows an enlarged view of a connection port opening of a textile sleeve according to one form of the technology.
Fig.10 shows a bottom view of a textile sleeve according to one form of the technology.
Fig. 11 is a perspective view of a patient interface comprising a pair of textile sleeves according to one form of the present technology.
Fig. 12 is a perspective view of a textile sleeve according to one form of the present technology.
Fig. 13A is a schematic of an example knit pattern for a textile sleeve according to form of the present technology.
Fig. 13B is a schematic of an example weave pattern for a textile sleeve according to form of the present technology.
Fig. 13C is a schematic of another example weave pattern for a textile sleeve according to form of the present technology.
Fig. 14A shows a first rear perspective view of an end cap for a positioning and stabilising structure according to one form of the technology.
Fig. 14B shows a second rear perspective view of the end cap of Fig. 14A.
Fig. 14C shows a rear view of the end cap of Fig. 14A.
Fig. 14D shows a front view of the end cap of Fig. 14A.
Fig. 14E shows a front perspective view of the end cap of Fig. 14A.
Fig. 14F shows a side view of the end cap of Fig. 14A.
Fig. 14G shows an enlarged cross-section side view of the end cap of Fig. 14A.
Fig. 15 shows a cross-section of an end cap applied to a gas delivery tube and a textile sleeve of a positioning and stabilising structure according to one form of the technology.
Figs. 16A shows a top perspective view of an end cap for a positioning and stabilising structure according to one form of the technology.
Fig. 16B shows a top view of the end cap of Fig. 16A.
Figs. 17A shows a schematic of a cross-section through a positioning and stabilising structure having a high friction interface between a gas delivery tube and a textile sleeve according to one form of the technology.
Fig. 17B shows a schematic of a cross-section through another positioning and stabilising structure having a high friction interface between a gas delivery tube and a textile sleeve according to one form of the technology.
Fig. 18 shows a schematic of a cross-section through a positioning and stabilising structure having a double-sided tape applied between a gas delivery tube and a textile sleeve according to one form of the technology.
Fig. 19 shows a schematic of a cross-section through a positioning and stabilising structure having a moulded end portion applied to a gas delivery tube and a textile sleeve according to one form of the technology.
Fig. 20 shows a cross-section of another form of end cap applied to a gas delivery tube and a textile sleeve of a positioning and stabilising structure according to one form of the technology, wherein the gas delivery tube is connected to a plenum chamber of a patient interface.
Fig. 21 shows a schematic of a cross-section through a positioning and stabilising structure having seam tape provided over an end of a textile sleeve according to one form of the technology.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient, e.g. up to 30cmH₂O.

### 4.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs - the actual sealing surface - may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 4.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.2 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 4.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

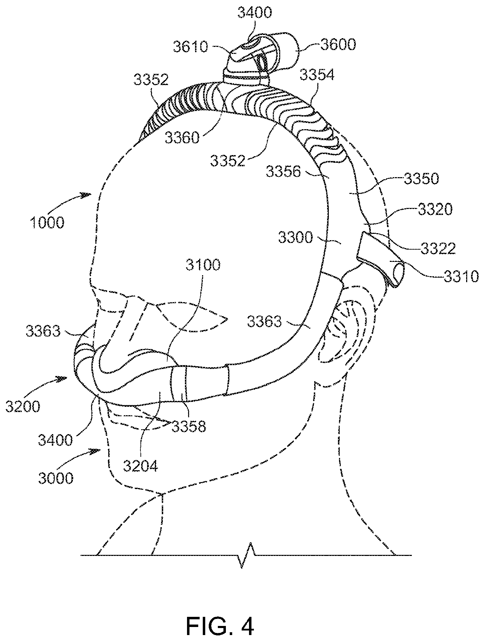

In some forms of the present technology, the positioning and stabilising structure 3300 comprises one or more gas delivery tubes 3350 that deliver pressurised air received from a conduit forming part of the air circuit 4170 from the RPT device to the patient's airways, for example as shown in Fig. 4.

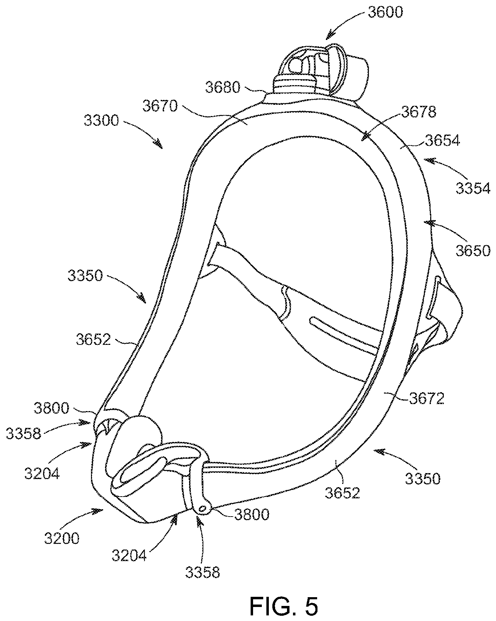

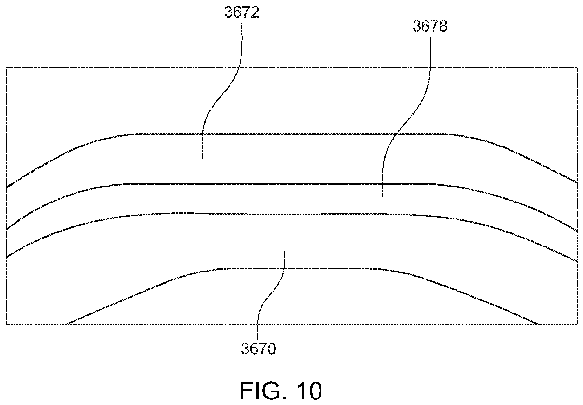

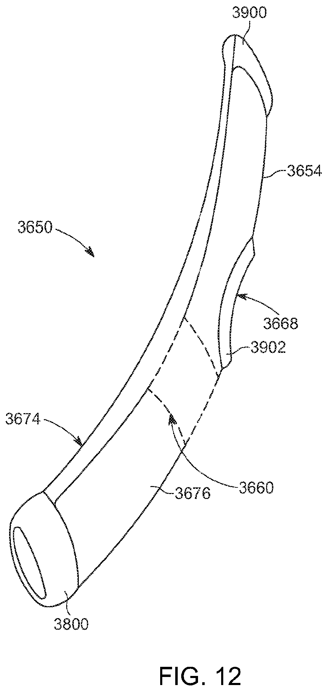

In examples, the positioning and stabilising structure 3300 comprises two tubes 3350 that deliver air to the seal-forming structure 3100 from the air circuit 4170. The tubes 3350 are an integral part of the positioning and stabilising structure 3300 of patient interface 3000 to position and stabilise the seal-forming structure 3100 of the patient interface to the appropriate part of the patient's face (for example, the nose or the nose and mouth). This allows the conduit of air circuit 4170 providing the flow of pressurised air to connect to a connection port 3600 of the patient interface in a position other than in front of the patient's face, which may be unsightly to some people. While the use of a pair of tubes 3350 has some advantages, in some examples the positioning and stabilising structure 3300 comprises only a single tube 3350 configured to overlie the patient's head on one side. A strap or other stabilising component may be provided to the other side of the patient's head between the top end of the single tube 3350 and the seal-forming structure 3100, to provide balanced forces on the seal-forming structure 3100. Except where specifically stated otherwise, any of the examples of positioning and stabilising structures described below with reference to Figures 5-21 may comprise a single tube 3350 or two tubes 3350.

In certain forms of the present technology, at least one of the gas delivery tubes 3350 is constructed and arranged to contact, in use, at least a region of the patient's head superior to an otobasion superior of the patient's head.

The patient interface 3000 may comprise a connection port 3600 located proximal a top, side or rear portion of a patient's head. For example, in the form of the present technology illustrated in Fig. 4, the connection port 3600 is located on top of the patient's head, e.g. overlying the parietal bone. In this example the patient interface 3000 comprises an elbow 3610 to which the connection port 3600 is provided. The elbow 3610 may swivel with respect to the positioning and stabilising structure 3300 in order to decouple movement of a conduit connected to the connection port 3600 from the positioning and stabilising structure 3300. The elbow 3610 may connect to a fluid connection opening 3360 in the headgear tubing 3350 or in a component to which the headgear tubing 3350 is connected. Additionally, or alternatively, a conduit connected to the connection port 3600 may swivel with respect to the elbow 3610. In the illustrated example, elbow 3610 comprises a swivelling conduit connector comprising the connection port 3600 to which a conduit of the air circuit 4170 is able to connect, such that the conduit can rotate about its longitudinal axis with respect to the elbow 3610. In some examples the air circuit 4170 may connect to the fluid connection opening 3360. The elbow 3610 may rotatably connect to the fluid connection opening 3360 or to a ring received in the fluid connection opening 3360.

In the example shown in Fig. 4. the two tubes 3350 are integrally formed and comprise a fluid connection opening 3360 to which the swivel elbow connects. In other examples, where separate tubes are used, they may be indirectly connected together, for example each may be connected to a T-shaped conduit having two conduit arms each fluidly connectable to the tubes 3350. The crown connector may comprise a third conduit arm. The connection port 3600 may comprise an elbow 3610 received at the centre of the crown connector 3360. The elbow 3610 may be configured to swivel.

In certain examples of the present technology, the tubes 3350 are configured to receive a strap 3310 (for example by provision of strap engaging portions such as tabs 3320) at locations superior to and proximate the patient's ears.

In examples, at least one of the gas delivery tubes 3350 comprises a variable length portion 3352. The variable length portion 3352 may be extendable, compressible, or both extendable and compressible from its "at rest" or natural length. In examples, the variable length portion 3352 comprises at least one corrugated or concertina portion 3354. In examples the gas delivery tube comprises a substantially constant length portion 3356 which extends between the variable length portion 3352 and the end 3358 of the gas delivery tube 3350 which connects to a plenum chamber connector 3204, one of which is provided at each lateral side of the plenum chamber 3200.

The example shown in Fig. 4 is provided with sleeves 3363 of the prior art.

### 4.3.3.1 Textile sleeve

Referring next to Figs. 5 to 21, in one form of the technology at least one elongate textile sleeve 3650 is provided over the or each gas delivery tube 3350. The sleeve 3650 is located on the gas delivery tube 3350 such that at least a portion of the sleeve 3650 is in contact with the patient's face, for example the patient's cheek, in use. In some examples the sleeve 3650 is configured to cover both gas delivery tubes of the positioning and stabilising structure 3300, but in other forms of the technology (not shown) separate sleeves 3650 may be provided for each gas delivery tube 3350.

In examples, the sleeve 3650 is located on the gas delivery tube 3350 such that a portion 3652 of the sleeve overlaps the substantially constant length portion 3356 of the gas delivery tube 3350 and a portion 3654 overlaps the variable length portion 3352 of the gas delivery tube. As such, while the portions 3356 and 3352 of the gas delivery tube 3350 are visible in Fig. 4, these portions are hidden by the sleeve 3650 in Figs. 5 to 21.

Referring next to Fig. 5 in particular, in examples, a wall 3666 of the sleeve 3650 is provided with an opening 3668. In examples the opening 3668 is sized such that the patient can view a portion of the gas delivery tube 3350 through the opening.

In one form of the technology the sleeve 3650 is configured for use with a gas delivery tube 3350 which is provided with strap engaging portions 3320 (typically integrally formed with the gas delivery tube) for engaging a strap (e.g. a back strap), for example in the form of a tab with loop or hook for engaging the strap. A tab 3320 may have an aperture 3322 through which the strap can be threaded for engagement.

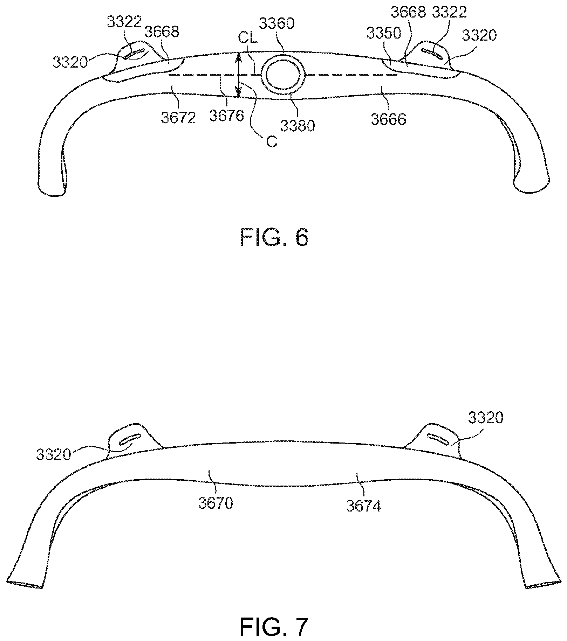

In an example, the sleeve 3650 is resiliently flexible in an axial direction. References herein to an axial direction are to the direction of the centreline CL of sleeve 3650, a portion of which is shown in Fig. 6. In examples the sleeve 3650 is resiliently flexible in a circumferential direction C. In one form of the technology the sleeve 3650 is flexible in both the axial and circumferential directions and has greater flexibility in the axial direction than the circumferential direction C.

The sleeve 3650 may comprise a first material 3670 and a second material 3672. The first material 3670 may form a patient contacting side 3674 of the sleeve 3650 and the second material 3672 may form a non-patient contacting side 3676 of the sleeve 3650. The first and second materials 3670, 3672 may be joined by seams 3678. In examples, at least one of the seams 3678 is configured to allow stretch in the circumferential direction. Examples of suitable seams may include pull-out seams, overlocker stitch seams and/or serger stitch seams.

The first material 3670 may be selected to provide good moisture wicking properties and/or soft hand feel. In one example the first material 3670 comprises a knit structure. In examples the first material 3670 is a dark colour. One example of a suitable material is Weimei JC1937A. The first material 3670 may be, for example, 0.6mm thick.

The second material 3672 may be selected to be smooth in order to reduce friction when sliding across bedding in use. One example of a suitable material is Gemmaknits IAP027AA. The second material 3672 may be, for example, 0.5mm thick. In examples both the first and second materials comprise nylon and spandex.

In some forms, the textile sleeve 3650 covers the entire length of the gas delivery tube 3350. This may be desirable for improving the aesthetic appearance of the positioning and stabilising structure 3300, and thus improving patient compliance with respiratory therapy. In other forms, the textile sleeve 3650 may cover only those portions of the gas delivery tube 3350 that are ordinarily in contact with the patient's skin during use, to improve patient comfort (by avoiding direct contact with the material of the gas delivery tube 3350, which is typically formed from silicone or like material).

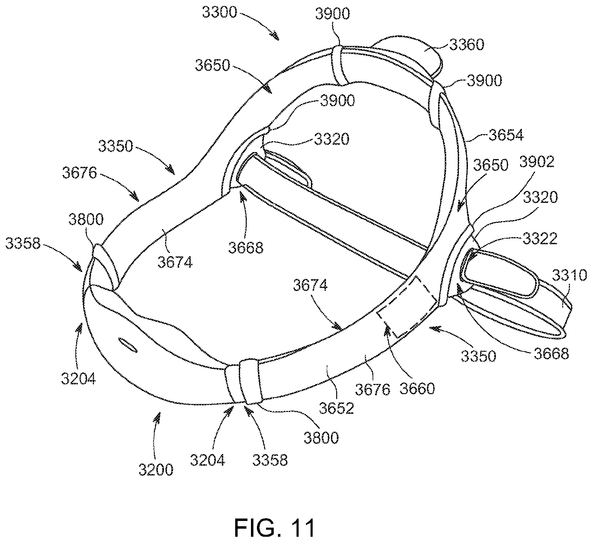

In some forms the textile sleeve 3650 is a substantially tubular structure comprising a single-piece knitted and/or woven structure that is resiliently flexible in both a circumferential direction C and an axial direction A (these directions being indicated in Fig. 11). The textile sleeve 3650 may comprise a single layer or more than one layer. The textile sleeve 3650 may be produced in a single knitting process. Where the textile sleeve comprises more than one layer, the layers may be interconnected during the single knitting process.

The circumferential resilient flexibility (across the width of the elongate textile sleeve 3650) enables the textile sleeve 3650 to form a snug fit with the gas delivery tube 3350, even if the gas delivery tube 3350 varies in width along its length. For example, in some forms of the present technology the gas delivery tube 3350 may be narrower at an end proximal to the fluid connection opening 3360 than at an end distal to the fluid connection opening 3360 and proximal to the plenum chamber connector 3204. In such cases the textile sleeve 3650, when fitted to the gas delivery tube 3350, will tend to contract to "hug" the tube 3350 at both a proximal portion 3654 and a distal portion 3652. Additionally, the circumferential resilient flexibility enables the sleeve 3650 to be stretched over a strap engaging portion of the gas delivery tube 3350, such as a hook tab 3320.

In some forms of the technology, the elasticity of the sleeve 3650 may vary along its length. For example, the elasticity may be greater in regions of the sleeve that are intended to accommodate wider sections of the gas delivery tube 3350, such as the sleeve portion 3654 which overlaps the variable length portion, or the tab 3320, than in regions that are intended to accommodate narrower sections of the gas delivery tube 3350, such as the sleeve portion 3652. The elasticity may be varied by varying the knitting density, the weave density, the number of loops in the knit, and/or the knit pattern or weave pattern itself, for example.

The axial resilient flexibility (along the length of the elongate textile sleeve 3650) tends to reduce or eliminate the likelihood of the sleeve 3650 wrinkling when the positioning and stabilising structure 3300 is worn by a patient and the gas delivery tube 3350 flexes as the patient moves.

By forming the textile sleeve 3650 with a single-piece knitted and/or woven structure, it is possible to provide a seamless, and therefore more comfortable, structure, without any sharp edges, and to minimise or eliminate post-processing steps that would otherwise be required, for example if the sleeve is made by cutting and joining sections of the material that is to be used to form the sleeve.

Textile sleeves 3650 such as those shown in any of Figs. 5-12 may be formed using a programmable knitting machine or weaving machine, such as an electronic double needle bed warp knitting machine or needle weaving machine. The use of such machines enables various aesthetic and functional properties to be imparted to the textile sleeve 3650 in a single production process.

For example, where a weaving machine is used, the weave can be programmed such that various pattern configurations, such as rearrangements of warp and weft twill weaves, can be imparted to provide comfort for skin contact and a more aesthetically pleasing finish.

Where a knitting machine is used, different knit patterns can be programmed for varying elasticity, softness, and other physical properties of the textile sleeve 3650. This may be done for both single-layer and multi-layer sleeves 3650. For a multi-layer sleeve 3650, the different layers may be differently constructed for robustness, hand-feel, or stretch by programming the knitting machine to use different yarn parameters, such as different yarn types, yarn density, yarn composition and the like. For a single-layer sleeve 3650, the yarn parameters may be varied in different regions along the length of the textile sleeve 3650, for example.

In some forms, the textile sleeve 3650 is at least partly formed from a first synthetic yarn, which may comprise, for example, fibres of nylon 66, polyester, acrylic and/or polyolefin.

The yarn specification may be selected to achieve various advantages, such as softness, surface smoothness and flatness, stretchability and/or translucency. For instance, relatively lower denier yarn may have increased translucency, thereby allowing the patient to see through the textile sleeve 3650. In addition, relatively low denier yarn may have increased softness, which achieves greater comfort for the patient. Alternatively, using relatively higher denier yarn may lead to ease of manufacture. The first synthetic yarn may have a denier in the range from about 20D to 80D, or about 25D to 75D, or about 30D to 70D, or about 35D to 65D, or about 40D to 60D, or about 45D to 55D.

The textile sleeve 3650 may alternatively, or additionally, be at least partly formed from a second synthetic yarn, such as yarn formed from fibres of elastane, Lycra^{™}, Spandex^{™} or ROICA^{™}. The second synthetic yarn may be a high-stretch elastomeric yarn having a denier in the range from about 50D to 140D, or about 55D to 135D, or about 60D to 130D, or about 65D to 125D, or about 70D to 120D, or about 75D to 115D, or about 80D to 110D, or about 85D to 105D, or about 90D to 100D. The high-stretch yarn may contribute to the circumferential resilient flexibility and/or the axial resilient flexibility of the textile sleeve 3650.

The textile sleeve may alternatively, or additionally, be at least partly formed from a second synthetic yarn, such as a yarn formed from fibres of elastane, Lycra^{™}, Spandex^{™} or ROICA^{™}. The second synthetic yarn may be a high stretch elastomeric yarn having a denier in the range from about 50D to 140D, or about 55D to 135D, or about 60D to 130D, or about 65D to 125D, or about 70D to 120D, or about 75D to 115D, or about 80D to 110D, or about 85D to 105D, or about 90D to 100D. The high-stretch yarn may contribute to the circumferential resilient flexibility and/or the axial resilient flexibility of the textile sleeve 3650.

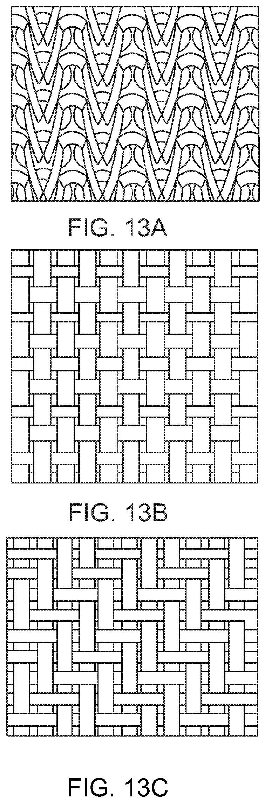

In some forms, the textile sleeve 3650 may be at least partly formed by one of, or a combination of any two or more of, a double-knitted (double-jersey) structure, such as shown in Fig. 13A; a plain weaving 1x1 structure, such as shown in Fig. 13B; and a plain weaving 2x2 structure, such as shown in Fig. 13C.

The textile sleeve 3650 may have a stretch (or stretchability) of more than 100% (such as about 120% to 300%) of its resting length or resting width. In some forms, the stretch may be between about 130% and 190%; or between 140% and 180%; or between 150% and 170%. The stretch may be greater in one direction than another. For example, the stretch may be greater in the circumferential direction C than in the axial direction A.

In some forms, the textile sleeve 3650 may be stretchable such that it contracts to less than 100% of its resting length (such as about 80% of its resting length) or less than 100% of its resting width (such as 80% of its resting width). For instance, when the textile sleeve 3650 stretches to more than 100% of its resting length, its resting width may contract because of the straightening by tension of the loops in the knit.

In some forms, the textile sleeve 3650 comprises a main structure, and one or more functional zones knitted and/or woven into the main structure. Each functional zone may have one or more textile properties that are different than those of the main structure. For example, as shown in Fig. 11, the sleeve 3650 may comprise a functional zone 3660 that extends partially along a non-patient contacting side 3676 of the textile sleeve 3650, and that differs in textile properties from the remainder of the non-patient contacting side 3676 and a patient contacting side 3674 of the sleeve 3650.

In some forms, the functional zone 3660 may be a transparent or translucent zone through which at least a portion of the gas delivery tube 3350 is visible. This enables the patient to view the portion of the gas delivery tube 3350 when the positioning and stabilising structure is not in use, such that if dust or other particulate matter is observed inside the tube 3350, the patient can then be prompted to clean the tube 3350.

A transparent or translucent zone 3660 may be formed during manufacturing of the sleeve 3650 by knitting or weaving a section of the sleeve 3650 with a lower density yarn or combination of yarns, a lower density knit or weave pattern, or a yarn formed from a specific fibre type, such as a synthetic monofilament or multifilament yarn formed from a fibre with a flattened cross section. One suitable type of yarn is described in US Patent Publication No. 20040168479.

Although only a single transparent or translucent zone 3660 is shown in Fig. 11, it will be appreciated that multiple such zones may be formed along the length of the textile sleeve 3650. In some examples, the entirety of the non-patient contacting side 3676 of the textile sleeve 3650 may be made transparent or translucent.

In some forms of the present technology, one or more of the functional zones may be an enhanced comfort zone that improves patient comfort. For example, a substantial part (such as those parts of the sleeve 3650 that ordinarily contact the patient in use) or the entirety of the patient contacting side 3674 of the textile sleeve 3650 may be formed as an enhanced comfort zone. To this end, the enhanced comfort zone may comprise one or more textured yarns that provide a soft feeling, thermal comfort, and breathability. A textured yarn may be a flat-twisted structure comprising a plurality of fibres having different cross-sections, for example.

In some forms, at least one enhanced friction zone of the one or more of the functional zones may comprise twills for localized gripping at the at least one enhanced friction zone.

In some forms, one or more zones may be formed with weave patterns that achieve specific aesthetic effects, such as weave ribbon with full but offset blinding, or V-weave velvet ribbon for a smooth appearance.

In some forms, the textile sleeve 3650 may be formed as a low-friction structure to facilitate installation on the gas delivery tube 3350. For example, the yarn density and fibre cross section may be selected to provide a low-friction surface of the sleeve 3650.

In some forms, the textile sleeve 3650 may be provided as a separate component to the patient (e.g., as part of a kit, or as a replacement part) for the patient to fit to the gas delivery tube(s) 3350. The textile sleeve 3650 may carry indicia to convey to the patient the correct orientation for fitting the sleeve 3650 to the tube(s) 3350. Such indicia may take the form of text or graphics, and/or a colour and/or pattern that indicates that one side is the patient contacting side 3674 and the other side is the non-patient contacting side 3676.

In some examples, at least part of the textile sleeve 3650 is coated or impregnated with at least one material conferring functional and/or aesthetic properties. For example, the textile sleeve 3650 may be coated or impregnated (e.g., by printing, or by impregnating fibres of the yarn prior to knitting or weaving the textile sleeve 3650) with a "glow in the dark" material (e.g. a phosphorescent material or a luminescent material). In some examples, textile sleeve 3650 may be coated or impregnated with an anti-microbial composition to reduce or eliminate bad odours or discolouration due to sweat absorption or absorption of other contaminants during use.

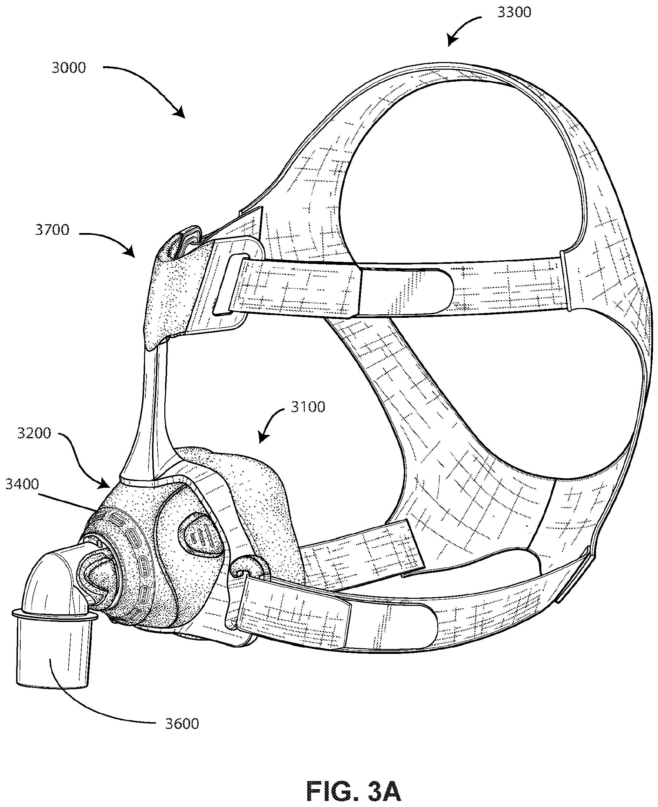

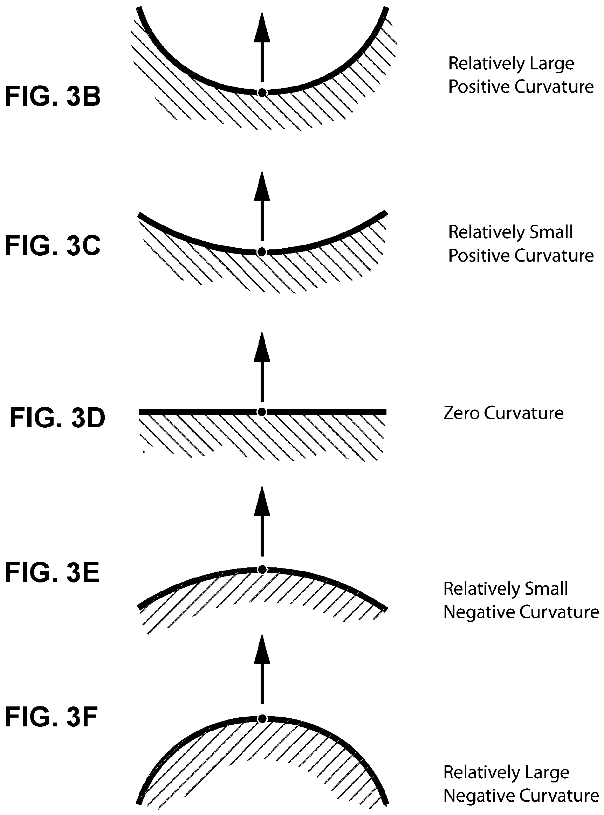

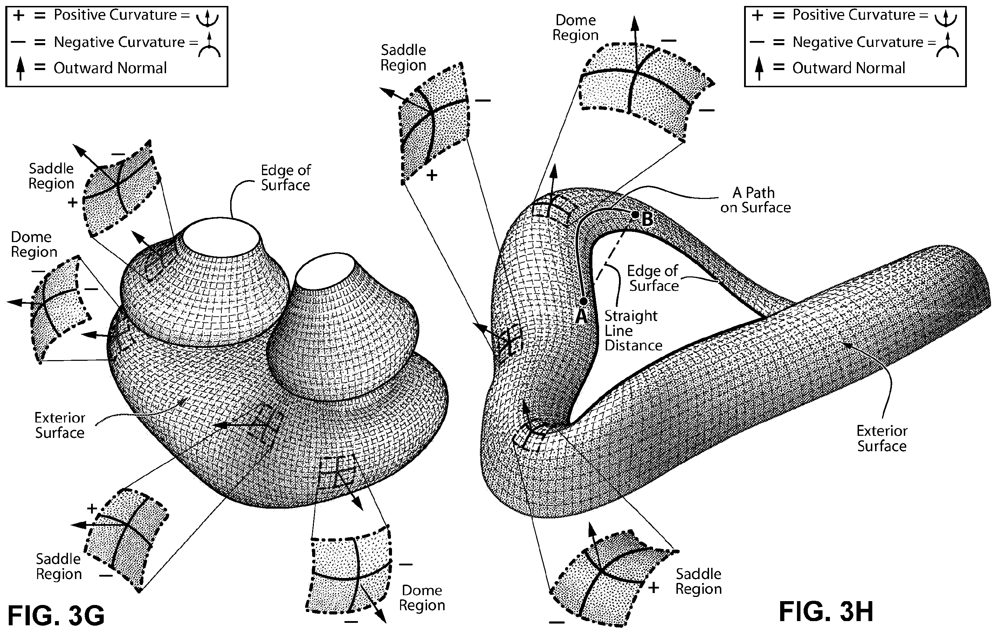

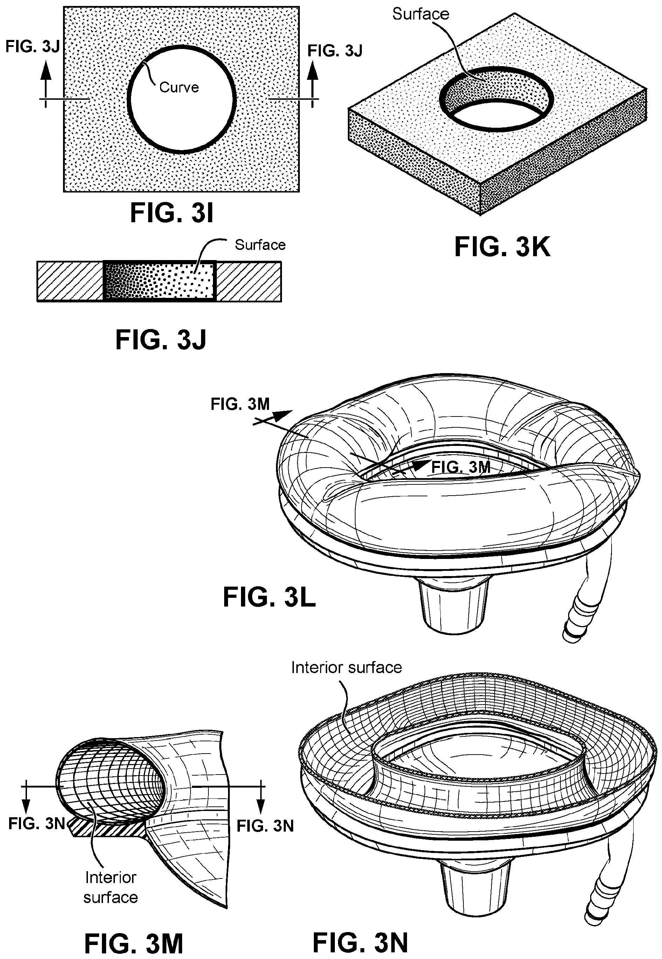

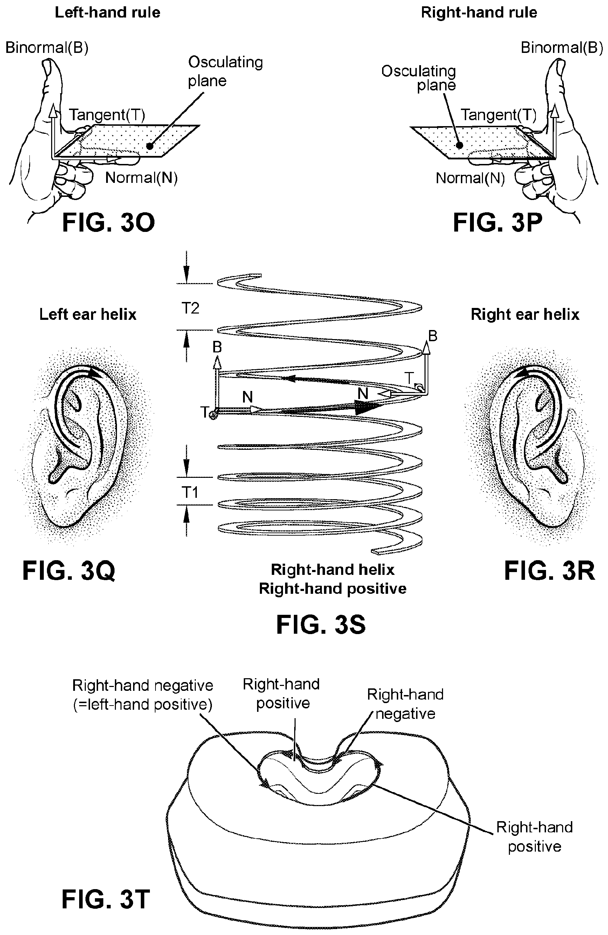

In one form of the technology, the sleeve 3650 is configured to allow the strap engaging portion 3320 to extend through the opening 3668. The opening 3668 may expose the entirety of the strap engaging portion 3320 as shown in Figs. 3, 6, 7, 8 and 11, or may expose just the aperture 3322 of the strap engaging portion 3320.

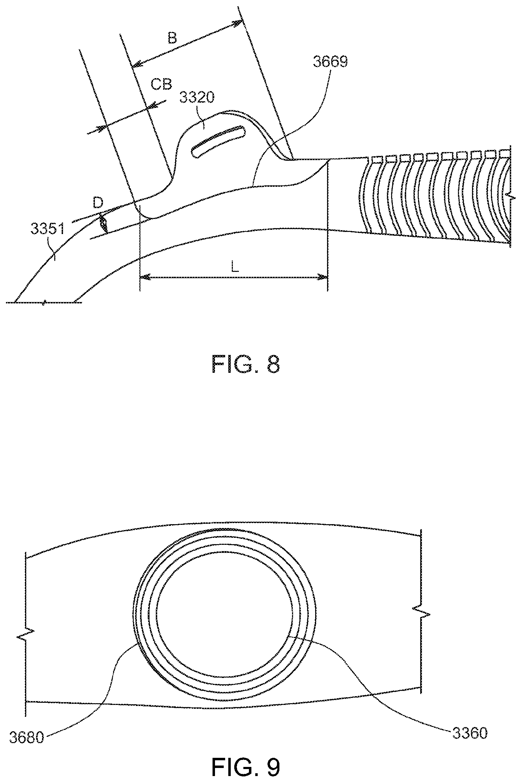

In some forms, the sleeve 3650 may comprise more than one such opening, for example if a single sleeve 3650 is arranged to extend over both gas delivery tubes 3350. In such cases, the sleeve 3650 may further comprise a connection port opening 3680 configured to allow an air circuit 4170 or an elbow 3600 to be connected to the connection port 3360, as best seen in Fig. 9.

Referring next to Fig. 8 in particular, in examples, the opening 3668 has a length L which is greater than the length B of the base of the strap engaging portion 3320, measured parallel to a longitudinal or central axis of the gas delivery tube 3350. In examples, the opening 3668 has a width which is greater than the width of the base of the strap engaging portion 3320, measured orthogonal to a longitudinal or central axis of the gas delivery tube 3350. In examples a clearance CB between the base of the strap engaging portion 3320 and the edge 3669 of the opening 3668 is at least sufficient to allow a patient to grasp the textile material surrounding the opening 3668 (for example between a finger and thumb in a pinching action) and to pull at least the material at the edge 3669 of the opening 3668 away from the gas delivery tube 3350, to thereby to allow the patient to view a portion of the tube 3350 which would otherwise be covered by the sleeve 3650. This may allow the patient to visually confirm the cleanliness of the gas delivery tube 3350 without removing the textile sleeve from the tube.

The opening 3668 may also have sufficient clearance CB from the base of the strap engaging portion 3320 to ensure that the gas delivery tube 3350 can move (e.g stretch) within the sleeve 3650 (for example when the variable length portion is extended) without the movement being restricted by the strap engaging portion 3320 abutting the edge 3669 of the opening 3668.

In one form of the technology such a clearance CB is provided at both ends of the opening 3668.

In one example the opening 3668 is substantially rectangular. In one example a substantially rectangular opening 3668 has arcuate corners. In other examples the opening 3668 is substantially stadium shaped - that is, it has a pair of parallel sides joined at either end by arcs or semicircles.

In one form of the technology the width of the opening 3668 is less than 50% of the circumference of the gas delivery tube 3350, when both measurements are taken at the same longitudinal position (e.g the same position on the centreline CL). This ensures that the material at the edge of the opening 3668 stays in contact with the gas delivery tube 3350. A wider opening may result in the material at the edge 3669 of the opening 3668 peeling back from the gas delivery tube 3350.

In one example the opening 3668 is configured to extend a distance D substantially 7-8 mm from the edge 3351 of the gas delivery tube 3350 which the strap engaging portion 3320 extends from, as shown in Fig. 8. This edge 3351 may be the bottom edge of the tube 3350 when the positioning and stabilising structure is in use. In examples, the opening is substantially entirely on the non-patient contacting side of the textile sleeve 3650, to avoid or prevent contact between the patient's skin and the surface of the gas delivery tube 3350.

In one example the opening 3668 may have a length L of substantially 65mm. The axial clearance distance CB may be substantially 5mm. The width of the opening may be selected such that the opening fits closely around the sides of the base of the strap engaging portion, e.g. the clearance distance D is less than 5mm, e.g. less than 2mm, e.g. close to or equal to zero. This narrower clearance D may result in a more integrated appearance, and may assist in ensuring the sleeve 3650 does not become wrinkled, creased or baggy over time.

In other examples the length L and distance CB may vary from those values provided above. For example, these values may vary to accommodate strap engaging portions of different length and/or width, or to provide increased or decreased clearance. The length L may be less than 65mm in examples.

In one form of the technology the sleeve 3650 extends over substantially the entire length of the positioning and stabilising structure, e.g. over both gas delivery tubes 3350 (as shown in Figs. 5-7). In examples, the positioning and stabilising structure comprises two strap engaging portions 3320 and the sleeve 3650 comprises two of the openings 3668 described above. In examples in which the sleeve 3650 extends over substantially the entire length of the positioning and stabilising structure, the sleeve 3650 may further be provided with a connection port opening 3680 configured to allow an air circuit 4170 or an elbow 3600 to be connected to a fluid connection opening 3360 of the gas delivery tube 3350, in use. The connection port opening 3680 may be a substantially circular opening and may be sized to provide a clearance around the fluid connection opening 3360 to allow the air circuit or elbow to be connected to the fluid connection opening 3360 without interfering with sleeve 3650.

In examples the ends of the sleeve 3650 and/or the material surrounding the edge of each opening 3668, 3680 may be provided with seam tape to reduce or eliminate fraying of the material.

In some examples, the opening 3668 may be formed in a single manufacturing process as part of the single-piece knitted and/or woven structure of the sleeve 3650, such that post-processing to create the opening 3668 is not required.

As shown in Fig. 11, in some forms, the opening 3668 may comprise an edge reinforcement structure 3902 to reduce or eliminate fraying of the textile sleeve 3650 in the region of the opening 3668 and the connection port opening 3680. The edge reinforcement structure 3902 may be formed by ultrasonic welding, laser cutting, application of a reinforcing seam tape, stitching, heat bonding, or deposition of additional material, such as a thermoplastic material.

In one example, seam tape is connected to an exterior surface of the textile sleeve 3650 by a suitable technique such as gluing. The opening 3668 may then be created in the seam tape and the textile sleeve 3650 at the same time.

A similar technique may be used to apply seam tape around the connection port opening 3680. In examples, the seam tape is applied to an internal surface of the textile sleeve 3650. However, in alternative examples the seam tape is applied to the exterior surface of the sleeve 3650.

### 4.3.3.2 Securing textile sleeve relative to gas delivery tube

As shown in Fig, 11, in some examples, the textile sleeve 3650 may also comprise an edge reinforcement structure 3800 at a first end proximal to the plenum chamber 3200, and an edge reinforcement structure 3900 at a second end, opposed to the first end, distal to the plenum chamber 3200. The edge reinforcement structure 3800 and/or the edge reinforcement structure 3900 may be formed by ultrasonic welding, laser cutting, application of a reinforcing seam tape, stitching, heat bonding, or deposition of additional material, such as a thermoplastic material.

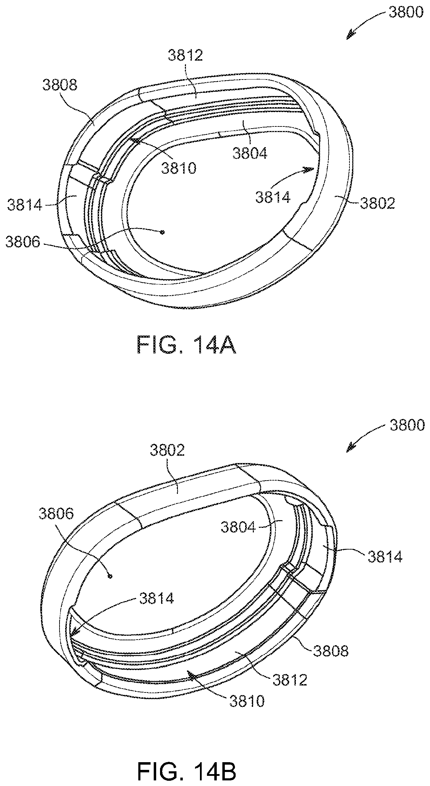

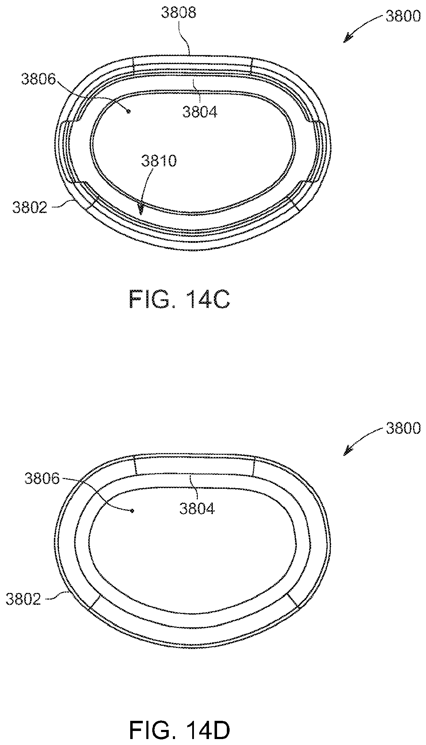

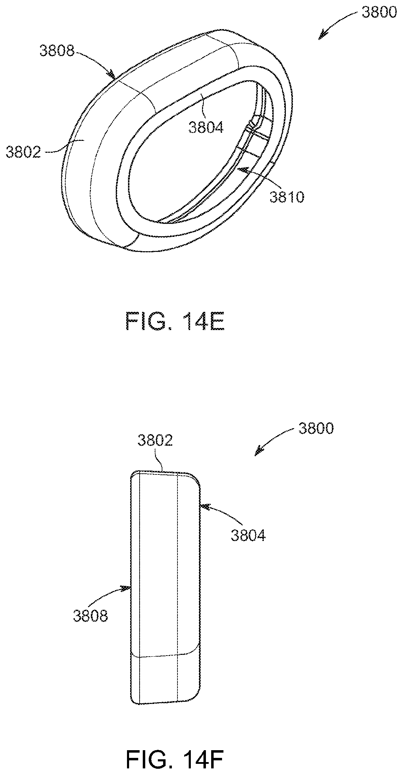

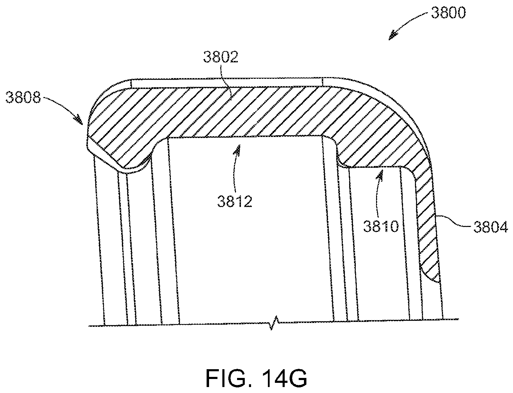

In examples, the textile sleeve 3650 may also be secured to the gas delivery tube 3350 proximal to the connection port 3600, i.e. at, or close to, the fluid connection opening 3360. In such examples, the edge reinforcement structure 3800 may be an end cap, such as that shown in Figs. 14A-14G.

In certain forms of the present technology, the textile sleeve 3650 may be secured relative to the gas delivery tubes 3350 at a position proximal to the ends 3358.

Securing the sleeve in this way may assist with providing stability to the relationship between the textile sleeve 3650 and gas delivery tubes 3350, particularly during dynamic movements where the patient may force the textile sleeve 3650 one way and the seal-forming structure 3100 or plenum chamber 3200 the other. In such cases, it is desirable for the textile sleeve 3650 to remain in place, and not come away from the connection between the gas delivery tubes 3350 and plenum chamber 3200 (for example, by rolling or riding up). While the relative dimensions of the textile sleeve 3650 and gas delivery tubes 3350 may be used to provide a tight fit to resist such movement, the introduction of a lubricant (for example, a washing detergent) may disrupt this, and it is desirable to provide a more secure connection for at least this reason.

According to one aspect of the present technology, end caps 3800 are provided at the ends 3358 of the gas delivery tubes 3350 which join the textile sleeve 3650 to the gas delivery tubes 3350. Fig. 5 shows one such example.

Figs. 14A-14G illustrate an exemplary end cap 3800. The end cap 3800 comprises an annular side wall 3802. As used herein, the term "annular" should be understood as meaning generally ring shaped, as opposed to necessitating strict adherence to a circular form - i.e. the side wall 3802 may be in the shape of an irregular annulus. An end flange 3804 extends radially inwardly from the side wall 3802, defining an end cap opening 3806 through which a plenum chamber connector 3204 may be received, in use.

In examples, the side wall 3802 has an end 3808 distal from the end flange 3804, and an inner surface 3810 between the end 3808 and the end flange 3804. The side wall 3802 may have at least one annular adhesive recess 3812, provided in the inner surface 3810. The adhesive recess 3812 may be used to assist dispensing of adhesive about the end cap 3800, and controlling the spread of same, before application to the textile sleeve 3650 and gas delivery tube 3350. In the example of Fig. 14A-14G, the adhesive recess 3812 is offset from both the end 3808 of the side wall 3802, and the end flange 3804. In an alternative example, the adhesive recess 3812 may extend to the end 3808 of the side wall 3802. In another alternative example, the adhesive recess 3812 may extend to the end flange 3804.

In examples, the side wall 3802 may have at least one axial recess 3814 extending from the end 3808 of the side wall 3802 towards the end flange 3804. The axial recess 3814 may be used to accommodate a seam 3678 of the textile sleeve 3650. In the example of Fig. 14A-14G, the end cap 3814 includes opposing axial recesses 3814 in order to accommodate two seams 3678 of the textile sleeve 3650 shown in Fig. 5.

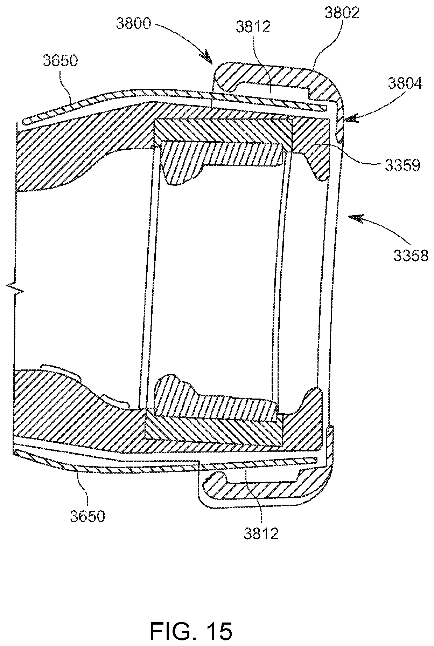

In examples, the end cap 3800 may be secured to the textile sleeve 3650 and gas delivery tube 3350 using an adhesive, for example a silicone. As shown in Fig. 15, the textile sleeve 3650 may be provided around the gas delivery tube 3350, proximate the end 3358 of the gas delivery tube 3350. The adhesive is applied to the adhesive recess 3812, and the end cap 3800 is fitted to the end 3358 of the gas delivery tube 3350. When fitted, the exemplary end cap 3800 extends from an outer surface of the textile sleeve 3650, over an end of the textile sleeve 3650, and over a portion of a rim 3359 (i.e. the end surface of the gas delivery tube 3350) of the gas delivery tube 3350. The overlapping portion of the end flange 3804 may assist with controlling spread of the adhesive. The overlapping portion of the end flange 3804 may act as a stop to assist with alignment during fitting of the end cap 3800.

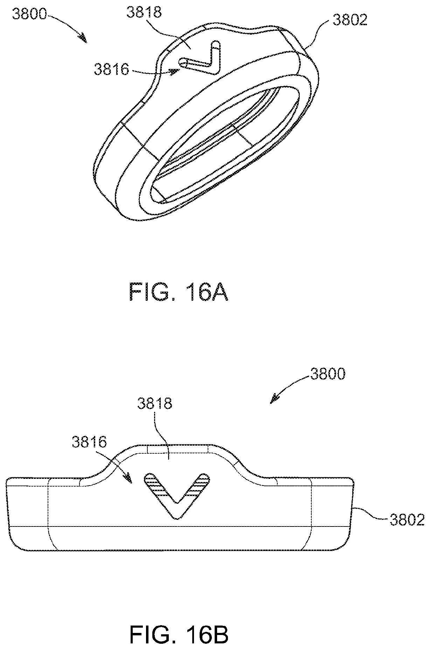

In examples, as shown in Fig. 16A and 16B, the end cap 3800 may have at least one visual indicator 3816 for use in indicating, for example, intended alignment of a gas delivery tube 3350 relative to plenum chamber connectors 3204, or a size of the positioning and stabilising structure 3300. In the example shown, the visual indicator 3816 is formed in the side wall 3802 of the end cap 3800. The side wall 3802 may have an extended tab 3818 to accommodate the visual indicator 3816.

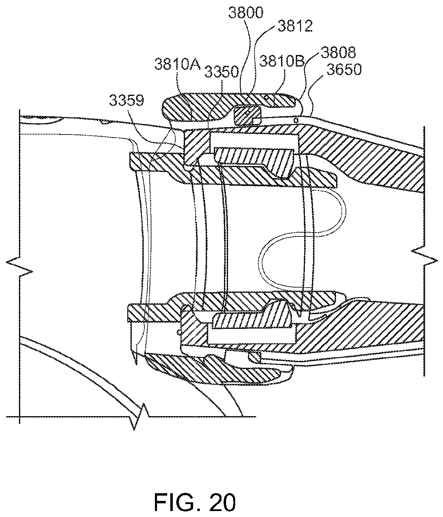

In another example of the technology, shown in Fig. 20, an end cap is provided which does not have an end flange 3804. In this example, the textile sleeve 3650 extends only as far as the adhesive recess 3812, but does not extend beyond the adhesive recess 3812 in the direction of the rim 3359 (i.e. the end of the tube 3350).

The inner surface 3810 has a first inner surface portion 3810A which extends between the adhesive recess 3812 and the end of the tube 3350, and a second inner surface portion 3810B which extends between the end 3808 and the adhesive recess.

The first inner surface portion 3810A is dimensioned to fit closely to tube 3350, while the second inner surface portion 3810B is dimensioned to accommodate the sleeve 3650 and is therefore wider.

The fit of the inner surface portion 3810A to the tube 3350 ensures that adhesive does not leak out the end of the end cap 3800 during manufacture.

In this example, adhesive provided to the adhesive recess 3812 bonds the sleeve 3650 to the tube 3350 and bonds the end cap 3800 to the tube 3350 and the sleeve 3650.

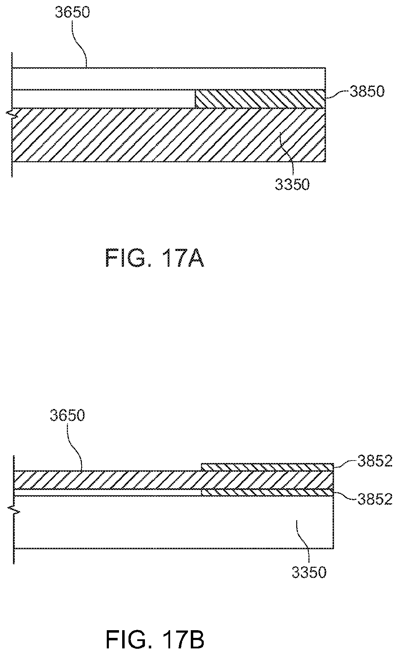

According to another aspect of the present technology, the textile sleeve 3650 is secured relative to the gas delivery tube 3350 by a localised high friction interface. At the localised high friction interface, relative movement between the textile sleeve 3650 and the gas delivery tube 3350 is restricted by an elevated degree of friction therebetween. It is envisaged that this may be achieved by providing a localised region on the textile sleeve 3650 which has different material characteristics, although in alternative examples the localised region may be provided on the gas delivery tube 3350. In the example of Fig. 17A, the localised high friction interface is provided by a discrete polymer layer 3850 between the textile sleeve 3650 and the gas delivery tube 3350. In examples, the discrete polymer layer 3850 may be provided by a thermoplastic polyurethane (TPU) tape, or a silicone tape. In examples, the silicone tape may be applied to inner and outer surfaces of the textile sleeve 3650.

In an alternative example, the discrete polymer layer 3850 may be provided by applying (for example, by screen printing) an uncured silicone layer onto the textile sleeve 3650, and curing the silicone to achieve the desired characteristics. In the example shown in Fig 17B, a silicone layer 3852 may be printed on inner and outer surfaces of the textile sleeve 3650.

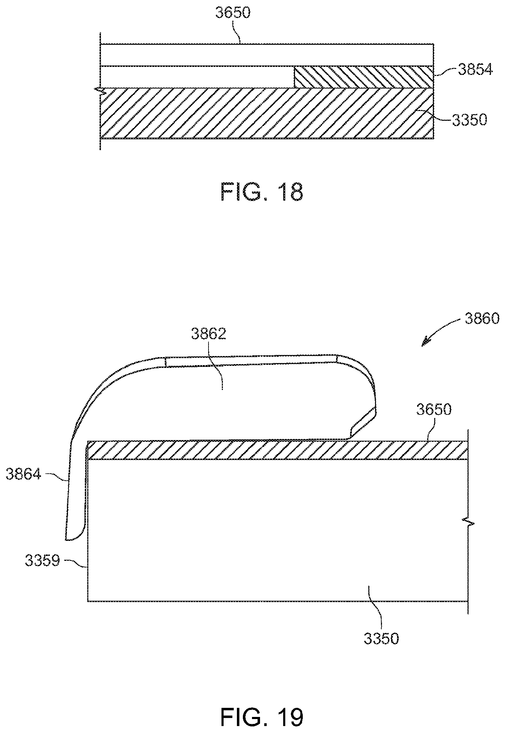

According to another aspect of the present technology, as shown in Figure 18, the textile sleeve 3650 may be secured relative to the gas delivery tube 3350 by a double-sided tape 3854 between the textile sleeve 3650 and the gas delivery tube 3350. In an example, the double-sided tape 3854 may be coated with a silicone adhesive on a first side facing the gas delivery tube 3350, and a non-silicone adhesive (for example, an acrylic adhesive) on a second side facing the textile sleeve 3650. One example of a suitable double-sided tape may be "Double-coated adhesive tape for silicone rubber bonding No. 5303W" supplied by Nitto Denko Corporation. Another example of a suitable double-sided tape may be "adt-x" adhesive tape supplied by VVB-Birzer Flächenschutz GmbH. It is envisaged that the double sided tape 3854 may be applied to the gas delivery tube 3350 before the textile sleeve 3650 is pulled or rolled into place.

According to another aspect of the present technology, as shown in Figure 19, the textile sleeve 3650 may be secured relative to the gas delivery tube 3350 by an overmoulded end portion 3860. The overmoulded end portion 3860 comprises an annular side wall 3862 extending along an outer surface of the textile sleeve 3650, and an end flange 3864 extending radially inwardly from the side wall 3862 over an end of the textile sleeve 3650 and over a portion of the rim 3359 of the gas delivery tube 3350. In alternative examples, the overmoulded end portion 3860 extends over the rim 3359 and into the interior of the gas delivery tube 3350. In examples, the overmoulded end portion 3860 may be made of a high durability silicone.

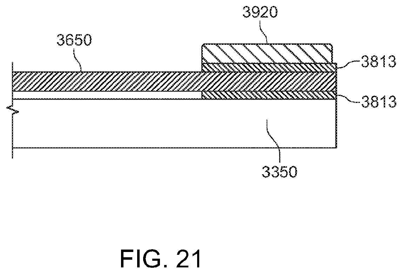

In another form of the technology, shown in Fig. 21, the textile sleeve 3650 may extend to the end of the tube 3350 and may be connected to the end of the tube 3350 by a layer of adhesive 3813. A layer of seam tape 3920 may be provided to the end of the tube 3350, over the textile sleeve 3650. The seam tape 3920 may be secured to the textile sleeve 3650 by a second layer of adhesive 3813.

In examples, the end of the textile sleeve 3650 may be provided with a notch (not shown). The notch may make it easier to roll the end of the sleeve 3650 back prior to depositing the layer of adhesive on the tube 3350. However, in alternative examples this notch may not be necessary.

### 4.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 4.3.5 Decoupling structure(s)

In one form the patient interface 3000 includes at least one decoupling structure, for example, a swivel or a ball and socket.

### 4.3.6 Connection port

Connection port 3600 allows for connection to the air circuit 4170.

### 4.3.7 Forehead support

In one form, the patient interface 3000 includes a forehead support 3700.

### 4.3.8 Anti-asphyxia valve

In one form, the patient interface 3000 includes an anti-asphyxia valve.

### 4.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.4 RPT DEVICE

An RPT device 4000 in accordance with one aspect of the present technology comprises mechanical, pneumatic, and/or electrical components and is configured to execute one or more algorithms, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

### 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers. such as temperature sensors. In one form, the heated wire circuit may be helically wound around the axis of the air circuit 4170. The heating element may be in communication with a controller such as a central controller. One example of an air circuit 4170 comprising a heated wire circuit is described in United States Patent 8733349.

### 4.6 HUMIDIFIER

### 4.6.1 Humidifier overview

In one form of the present technology there is provided a humidifier 5000 to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir, a humidifier inlet to receive a flow of air, and a humidifier outlet to deliver a humidified flow of air.

### 4.7 RESPIRATORY THERAPY MODES

Various respiratory therapy modes may be implemented by the disclosed respiratory therapy system, including CPAP and bi-level therapy.

### 4.8 GLOSSARY

For the purposes of the present technology disclosure, in certain forms of the present technology, one or more of the following definitions may apply. In other forms of the present technology, alternative definitions may apply.

### 4.8.1 General

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Patient*: A person, whether or not they are suffering from a respiratory condition.

*Pressure*: Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT)*: The application of a supply of air to an entrance to the airways at a treatment pressure that is typically positive with respect to atmosphere.

*Ventilator*: A mechanical device that provides pressure support to a patient to perform some or all of the work of breathing.

### 4.8.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate*: a thermoplastic polymer of Bisphenol-A Carbonate.

### 4.8.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component*: The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component*: A structure or component that will change shape, e.g. bend, when caused to support its own weight, within a relatively short period of time such as 1 second.

*Rigid structure or component*: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.8.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle:* The ratio of inhalation time, *Ti* to total breath time, *Ttot.*

*Effort (breathing)*: The work done by a spontaneously breathing person attempting to breathe.

*Expiratory portion of a breathing cycle*: The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) *Flattened*: Having a rise followed by a relatively flat portion, followed by a fall.
(ii) *M-shaped*: Having two local peaks, one at the leading edge, and one at the trailing edge, and a relatively flat portion between the two peaks.
(iii) *Chair-shaped*: Having a single local peak, the peak being at the leading edge, followed by a relatively flat portion.
(iv) *Reverse-chair shaped*: Having a relatively flat portion followed by single local peak, the peak being at the trailing edge.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle*: The period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate (Qpeak)*: The maximum value of flow rate during the inspiratory portion of the respiratory flow waveform.

*Respiratory flow rate,* patient *airflow rate, respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt)*: The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti)*: The duration of the inspiratory portion of the respiratory flow rate waveform.

*(exhalation) Time (Te)*: The duration of the expiratory portion of the respiratory flow rate waveform.

*(total) Time (Ttot)*: The total duration between the start of one inspiratory portion of a respiratory flow rate waveform and the start of the following inspiratory portion of the respiratory flow rate waveform.

*Typical recent ventilation*: The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.8.3 Ventilation

*Adaptive Servo-Ventilator (ASV)*: A servo-ventilator that has a changeable, rather than fixed target ventilation. The changeable target ventilation may be learned from some characteristic of the patient, for example, a respiratory characteristic of the patient.

*Backup rate*: A parameter of a ventilator that establishes the minimum breathing rate (typically in number of breaths per minute) that the ventilator will deliver to the patient, if not triggered by spontaneous respiratory effort.

*Cycled*: The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP)*: a base pressure, to which a pressure varying within the breath is added to produce the desired interface pressure which the ventilator will attempt to achieve at a given time.

*End expiratory pressure (EEP)*: Desired interface pressure which the ventilator will attempt to achieve at the end of the expiratory portion of the breath. If the pressure waveform template Π(Φ) is zero-valued at the end of expiration, i.e. Π(Φ) = 0 when Φ = 1, the EEP is equal to the EPAP.

*Inspiratory positive airway pressure (IPAP)*: Maximum desired interface pressure which the ventilator will attempt to achieve during the inspiratory portion of the breath.

*Pressure support*: A number that is indicative of the increase in pressure during ventilator inspiration over that during ventilator expiration, and generally means the difference in pressure between the maximum value during inspiration and the base pressure (e.g., *PS = IPAP - EPAP*). In some contexts pressure support means the difference which the ventilator aims to achieve, rather than what it actually achieves.

*Servo-ventilator*: A ventilator that measures patient ventilation, has a target ventilation, and which adjusts the level of pressure support to bring the patient ventilation towards the target ventilation.

*Spontaneous*/*Timed (S*/*T)*: A mode of a ventilator or other device that attempts to detect the initiation of a breath of a spontaneously breathing patient. If however, the device is unable to detect a breath within a predetermined period of time, the device will automatically initiate delivery of the breath.

*Swing*: Equivalent term to pressure support.

*Triggered*: When a ventilator delivers a breath of *air* to a spontaneously breathing patient, it is said to be triggered to do so at the initiation of the respiratory portion of the breathing cycle by the patient's efforts.

### 4.8.4 Anatomy

### 4.8.4.1 Anatomy of the face

*Ala*: the external outer wall or "wing" of each nostril (plural: alar)
*Alare*: The most lateral point on the nasal *ala.*

*Alar curvature (or alar crest) point*: The most posterior point in the curved base line of each *ala,* found in the crease formed by the union of the *ala* with the cheek.

*Auricle*: The whole external visible part of the ear.

*(nose) Bony framework*: The bony framework of the nose comprises the nasal bones, the frontal process of the maxillae and the nasal part of the frontal bone.

*(nose) Cartilaginous framework*: The cartilaginous framework of the nose comprises the septal, lateral, major and minor cartilages.

*Columella*: the strip of skin that separates the nares and which runs from the pronasale to the upper lip.

*Columella angle*: The angle between the line drawn through the midpoint of the nostril aperture and a line drawn perpendicular to the Frankfort horizontal while intersecting subnasale.

*Frankfort horizontal plane*: A line extending from the most inferior point of the orbital margin to the left tragion. The tragion is the deepest point in the notch superior to the tragus of the auricle.

*Glabella*: Located on the soft tissue, the most prominent point in the midsagittal plane of the forehead.

*Lateral nasal cartilage*: A generally triangular plate of cartilage. Its superior margin is attached to the nasal bone and frontal process of the maxilla, and its inferior margin is connected to the greater alar cartilage.

*Lip, lower (labrale inferius)*:
*Lip, upper (labrale superius)*:
*Greater alar cartilage*: A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils)*: Approximately ellipsoidal apertures forming the entrance to the nasal cavity. The singular form of nares is naris (nostril). The nares are separated by the nasal septum.

*Naso-labial sulcus or Naso-labial fold*: The skin fold or groove that runs from each side of the nose to the corners of the mouth, separating the cheeks from the upper lip.

*Naso-labial angle*: The angle between the columella and the upper lip, while intersecting subnasale.

*Otobasion inferior*: The lowest point of attachment of the auricle to the skin of the face.

*Otobasion superior*: The highest point of attachment of the auricle to the skin of the face.

*Pronasale*: the most protruded point or tip of the nose, which can be identified in lateral view of the rest of the portion of the head.

*Philtrum*: the midline groove that runs from lower border of the nasal septum to the top of the lip in the upper lip region.

*Pogonion*: Located on the soft tissue, the most anterior midpoint of the chin.

*Ridge (nasal)*: The nasal ridge is the midline prominence of the nose, extending from the Sellion to the Pronasale.

*Sagittal plane*: A vertical plane that passes from anterior (front) to posterior (rear). The midsagittal plane is a sagittal plane that divides the body into right and left halves.

*Sellion*: Located on the soft tissue, the most concave point overlying the area of the frontonasal suture.

*Septal cartilage (nasal)*: The nasal septal cartilage forms part of the septum and divides the front part of the nasal cavity.

*Subalare*: The point at the lower margin of the alar base, where the alar base joins with the skin of the superior (upper) lip.

*Subnasal point*: Located on the soft tissue, the point at which the columella merges with the upper lip in the midsagittal plane.

*Supramenton*: The point of greatest concavity in the midline of the lower lip between labrale inferius and soft tissue pogonion

### 4.8.4.2 Anatomy of the skull

*Frontal bone*: The frontal bone includes a large vertical portion, the *squama frontalis,* corresponding to the region known as the forehead.

*Mandible*: The mandible forms the lower jaw. The mental protuberance is the bony protuberance of the jaw that forms the chin.

*Maxilla*: The maxilla forms the upper jaw and is located above the mandible and below the orbits. The *frontal process of the maxilla* projects upwards by the side of the nose, and forms part of its lateral boundary.

*Nasal bones*: The nasal bones are two small oblong bones, varying in size and form in different individuals; they are placed side by side at the middle and upper part of the face, and form, by their junction, the "bridge" of the nose.

*Nasion*: The intersection of the frontal bone and the two nasal bones, a depressed area directly between the eyes and superior to the bridge of the nose.

*Occipital bone*: The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit*: The bony cavity in the skull to contain the eyeball.

*Parietal bones*: The parietal bones are the bones that, when joined together, form the roof and sides of the cranium.

*Temporal bones*: The temporal bones are situated on the bases and sides of the skull, and support that part of the face known as the temple.

*Zygomatic bones*: The face includes two zygomatic bones, located in the upper and lateral parts of the face and forming the prominence of the cheek.

### 4.8.4.3 Anatomy of the respiratory system

*Diaphragm*: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx*: The larynx, or voice box houses the vocal folds and connects the inferior part of the pharynx (hypopharynx) with the trachea.

*Lungs*: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity*: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx*: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.8.5 Patient interface

*Anti-asphyxia valve (AAV)*: The component or sub-assembly of a mask system that, by opening to atmosphere in a failsafe manner, reduces the risk of excessive CO₂ rebreathing by a patient.

*Elbow*: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame*: Frame will be taken to mean a mask structure that bears the load of tension between two or more points of connection with a headgear. A mask frame may be a non-airtight load bearing structure in the mask. However, some forms of mask frame may also be air-tight.

*Headgear*: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane*: Membrane will be taken to mean a typically thin element that has, preferably, substantially no resistance to bending, but has resistance to being stretched.

*Plenum chamber*: a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal*: May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell*: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener*: A stiffener will be taken to mean a structural component designed to increase the bending resistance of another component in at least one direction.

*Strut*: A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent*: (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.8.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 4.8.6.1 Curvature in one dimension

The curvature of a plane curve at *p* may be described as having a sign (e.g. positive, negative) and a magnitude (e.g. 1/radius of a circle that just touches the curve at *p*).

*Positive curvature*: If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature*: If the curve at *p* is a straight line, the curvature will be taken to be zero (if the imaginary small person leaves the point *p*, they can walk on a level, neither up nor down). See Fig. 3D.

*Negative curvature*: If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 4.8.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions*: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface*: A connected set of points on a surface. The set of points in a region may have similar characteristics, e.g. curvatures or signs.

*Saddle region*: A region where at each point, the principal curvatures have opposite signs, that is, one is positive, and the other is negative (depending on the direction to which the imaginary person turns, they may walk uphill or downhill).

*Dome region*: A region where at each point the principal curvatures have the same sign, e.g. both positive (a "concave dome") or both negative (a "convex dome").

*Cylindrical region*: A region where one principal curvature is zero (or, for example, zero within manufacturing tolerances) and the other principal curvature is non-zero.

*Planar region*: A region of a surface where both of the principal curvatures are zero (or, for example, zero within manufacturing tolerances).

*Edge of a surface*: A boundary or limit of a surface or region.

*Path*: In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length*: In certain forms of the present technology, 'path length' will be taken to mean the distance along the surface from *f*(0) to f(1), that is, the distance along the path on the surface. There may be more than one path between two points on a surface and such paths may have different path lengths. (The path length for the imaginary person would be the distance they have to walk on the surface along the path).

*Straight-line distance*: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.8.6.3 Space curves

*Space curves*: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector)*: For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.8.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 4.9 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 4.10 REFERENCE SIGNS LIST

| | |
|---|---|
| patient | 1000 |
| bed partner | 1100 |
| patient interface | 3000 |
| seal forming structure | 3100 |
| plenum chamber | 3200 |
| plenum chamber connector | 3204 |
| positioning and stabilising structure | 3300 |
| strap | 3310 |
| strap engaging portion | 3320 |
| aperture in strap engaging portion | 3322 |
| gas delivery tube | 3350 |
| edge of tube | 3351 |
| variable length portion | 3352 |
| concertina portion | 3354 |
| constant length portion | 3356 |
| end | 3358 |
| rim | 3359 |
| fluid connection opening | 3360 |
| sleeve | 3363 |
| vent | 3400 |
| connection port | 3600 |
| elbow | 3610 |
| textile sleeve | 3650 |
| portion of sleeve | 3652 |
| portion of sleeve | 3654 |
| functional zone | 3660 |
| wall | 3666 |
| opening | 3668 |
| perimeter | 3669 |
| first material | 3670 |
| second material | 3672 |
| first (patient contacting) side of sleeve | 3674 |
| second (non-patient contacting) side of sleeve | 3676 |
| seam | 3678 |
| connection port opening | 3680 |
| forehead support | 3700 |
| end cap | 3800 |
| side wall | 3802 |
| end flange | 3804 |
| opening | 3806 |
| end | 3808 |
| inner surface | 3810 |
| adhesive recess | 3812 |
| adhesive | 3813 |
| axial recess | 3814 |
| visual indicator | 3816 |
| tab | 3818 |
| discrete polymer layer | 3850 |
| silicone layer | 3852 |
| double-sided tape | 3854 |
| overmoulded end portion | 3860 |
| side wall | 3862 |
| end flange | 3864 |
| edge reinforcement structure | 3900 |
| edge reinforcement structure | 3902 |
| seam tape | 3920 |
| RPT device | 4000 |
| air circuit | 4170 |
| humidifier | 5000 |
| length of opening | L |
| length of base of connection means | B |
| clearance | CB |
| centreline of sleeve | CL |
| circumferential direction | C |
| distance | D |

## Claims

1. A positioning and stabilising structure (3300) to provide a force to hold a seal-forming structure (3100) in a therapeutically effective position on a patient's head, the seal-forming structure (3100) constructed and arranged to form a seal with a region of the patient's face surrounding an entrance to the patient's airways for sealed delivery of a flow of air at a therapeutic pressure of at least 6 cmH₂O above ambient air pressure to at least the patient's nares throughout the patient's respiratory cycle in use, the positioning and stabilising structure (3300) comprising:
two gas delivery tubes (3350) to receive the flow of air from a connection port on top of the patient's head and to deliver the flow of air to the entrance of the patient's airways via the seal-forming structure (3100), the gas delivery tubes (3350) being constructed and arranged to contact, in use, at least a region of the patient's head superior to an otobasion superior of the patient's head,
the positioning and stabilising structure (3300) further comprising an elongate textile sleeve (3650) provided around the gas delivery tubes (3350) and arranged to be in contact, in use, with the patient's face, the sleeve (3650) comprising a wall (3666) with two openings (3668) therein for allowing the patient (1000) to view a portion of the gas delivery tubes (3350) when the positioning and stabilising structure (3300) is not in use,
wherein the width of each opening (3668) is less than 50% of the circumference of the gas delivery tubes (3350), when the opening (3668) and the tube (3350) are measured at the same longitudinal position,
wherein the gas delivery tubes (3350) comprise two strap engaging portions (3320) for engaging a strap (3310), and the textile sleeve (3650) is arranged such that the strap engaging portions (3320) protrude through each of the two respective openings (3668).

2. The positioning and stabilising structure (3300) of claim **1,** wherein the textile sleeve (3650) is resiliently flexible in an axial direction and a circumferential direction, and has a greater flexibility in the axial direction than the circumferential direction.

3. The positioning and stabilising structure (3300) of claim 1 or 2 wherein the openings (3668) are substantially oval, elliptical or stadium shaped.

4. The positioning and stabilising structure (3300) of any one of claims 1 to 3, wherein the gas delivery tubes (3350) comprise a concertina portion (3354) and a non-concertina portion, wherein the non-concertina portion is on the opposite side of the concertina portion (3354) to the connection port (3360, 3600), wherein the textile sleeve (3650) is arranged such that the entire edge of each of the openings (3668) lies over the non-concertina portion of the respective gas delivery tubes (3350).

5. The positioning and stabilising structure (3300) of claim 4 wherein the sleeve (3363, 3650) extends to the end (3358, 3808) of the non-concertina portion of the respective gas delivery tube (3350) which is opposite the concertina portion (3354).

6. The positioning and stabilising structure (3300) of any one of claims 1 to 5 wherein each of the two strap engaging portions (3320) comprises a tab.

7. The positioning and stabilising structure (3300) of any one of claims 1 to 6 wherein there is a clearance between the edge (3351, 3669) of each opening (3668) and the respective strap engaging portion (3320).

8. The positioning and stabilising structure (3300) of claim 7 wherein the clearance is in the longitudinal direction.

9. The positioning and stabilising structure (3300) of claim 8, wherein the clearance allows the gas delivery tubes (3350) to stretch, in use, without the respective strap engaging portion (3320) contacting the edge (3351, 3669) of the respective opening (3668).

10. The positioning and stabilising structure (3300) of any one of claims 1 to 9 wherein each of the two openings (3668) is substantially 65mm long.

11. The positioning and stabilising structure (3300) of any one of claims 7 to 10, when dependent on claim 6, wherein there is a lateral clearance between each tab and each lateral edge of the respective opening (3668) of less than 5mm.

12. The positioning and stabilising structure (3300) of any one of claims 1 to 11, wherein the textile sleeve (3650) comprises a first material (3670) on a patient contacting side (3674, 3676) of the sleeve (3363, 3650), and a second material (3672) on a non patient contacting side (3674, 3676) of the sleeve (3363, 3650).

13. The positioning and stabilising structure (3300) of claim 12, wherein the first material (3670) is connected to the second material (3672) by a seam (3678), wherein the seam (3678) is configured to allow stretch in the circumferential direction.

14. The positioning and stabilising structure (3300) of any one of claims 1 to 13, wherein seam (3678) tape is provided to the edge (3351, 3669) of the opening (3668) to reduce or eliminate fraying of the textile.

15. The positioning and stabilising structure (3300) of any one of claims 1 to 14, wherein the two tubes (3350) are integrally formed.

## Patentansprüche

1. Positionierungs- und Stabilisierungsstruktur (3300) zum Bereitstellen einer Kraft zum Halten einer dichtungsbildenden Struktur (3100) in einer therapeutisch wirksamen Stellung am Kopf eines Patienten, wobei die dichtungsbildende Struktur (3100) aufgebaut und eingerichtet ist, um im Gebrauch zur abgedichteten Zufuhr eines Stroms von Luft mit einem therapeutischen Druck von mindestens 6 cmH₂O über Umgebungsluftdruck während des gesamten Atemzyklus des Patienten zu mindestens den Nasenlöchern des Patienten eine Dichtung mit einem einen Eingang in die Atemwege des Patienten umgebenden Bereich des Gesichts des Patienten zu bilden, wobei die Positionierungs- und Stabilisierungsstruktur (3300) Folgendes aufweist:
zwei Gaszuführschläuche (3350) zum Aufnehmen des Stroms von Luft von einem Anschluss oben am Kopf des Patienten und zum Zuführen des Stroms von Luft zu dem Eingang der Atemwege des Patienten über die dichtungsbildende Struktur (3100), wobei die Gaszuführschläuche (3350) aufgebaut und eingerichtet sind, um sich im Gebrauch mit mindestens einem Bereich des Kopfs des Patienten oberhalb eines Otobasion superios des Kopfs des Patienten in Kontakt zu befinden,
wobei die Positionierungs- und Stabilisierungsvorrichtung (3300) ferner eine langgestreckte Textilhülle (3650) aufweist, die um die Gaszuführschläuche (3350) bereitgestellt ist und dazu eingerichtet ist, sich im Gebrauch mit dem Gesicht des Patienten in Kontakt zu befinden, wobei die Hülle (3650) eine Wand (3666) mit zwei Öffnungen (3668) darin aufweist, um dem Patienten (1000) zu ermöglichen, einen Abschnitt der Gaszuführschläuche (3350) zu betrachten, wenn die Positionierungs- und Stabilisierungsstruktur (3300) nicht in Gebrauch ist,
wobei die Breite jeder Öffnung (3668) geringer als 50 % des Umfangs der Gaszuführschläuche (3350) ist, wenn die Öffnung (3668) und der Schlauch (3350) an derselben Längsposition gemessen werden,
wobei die Gaszuführschläuche (3350) zwei Riemeneingriffsabschnitte (3320) zum Ineingriffnehmen eines Riemens (3310) aufweisen und die Textilhülle (3650) derart angeordnet ist, dass die Riemeneingriffsabschnitte (3320) jeweils durch eine jeweilige der zwei Öffnungen (3668) ragen.

2. Positionierungs- und Stabilisierungsstruktur (3300) nach Anspruch 1, wobei die Textilhülle (3650) in einer Axialrichtung und einer Umfangsrichtung elastisch biegsam ist und in der Axialrichtung eine höhere Biegsamkeit aufweist als in der Umfangsrichtung.

3. Positionierungs- und Stabilisierungsstruktur (3300) nach Anspruch 1 oder 2, wobei die Öffnungen (3668) im Wesentlichen oval, elliptisch oder stadiumförmig sind.

4. Positionierungs- und Stabilisierungsstruktur (3300) nach einem der Ansprüche 1 bis 3, wobei die Gaszuführschläuche (3350) einen Faltenbalgabschnitt (3354) und einen faltenbalglosen Abschnitt aufweisen, wobei sich der faltenbalglose Abschnitt auf der dem Anschluss (3360, 3600) gegenüberliegenden Seite des Faltenbalgabschnitts (3354) befindet, wobei die Textilhülle (3650) derart angeordnet ist, dass der gesamte Rand jeder der Öffnungen (3668) über dem faltenbalglosen Abschnitt der jeweiligen Gaszuführschläuche (3350) liegt.

5. Positionierungs- und Stabilisierungsstruktur (3300) nach Anspruch 4, wobei sich die Hülle (3363, 3650) zu dem Ende (3358, 3808) des faltenbalglosen Abschnitts des jeweiligen Gaszuführschlauchs (3350) erstreckt, der dem Faltenbalgabschnitt (3354) gegenüberliegt.

6. Positionierungs- und Stabilisierungsstruktur (3300) nach einem der Ansprüche 1 bis 5, wobei die zwei Riemeneingriffsabschnitte (3320) jeweils eine Lasche aufweisen.

7. Positionierungs- und Stabilisierungsstruktur (3300) nach einem der Ansprüche 1 bis 6, wobei zwischen dem Rand (3351, 3669) jeder Öffnung (3668) und dem jeweiligen Riemeneingriffsabschnitt (3320) ein Abstand vorhanden ist.

8. Positionierungs- und Stabilisierungsstruktur (3300) nach Anspruch 7, wobei der Abstand in der Längsrichtung vorhanden ist.

9. Positionierungs- und Stabilisierungsstruktur (3300) nach Anspruch 8, wobei der Abstand ermöglicht, dass sich die Gaszuführschläuche (3350) im Gebrauch dehnen, ohne dass der jeweilige Riemeneingriffsabschnitt (3320) mit dem Rand (3351, 3669) der jeweiligen Öffnung (3668) in Kontakt kommt.

10. Positionierungs- und Stabilisierungsstruktur (3300) nach einem der Ansprüche 1 bis 9, wobei die zwei Öffnungen (3668) jeweils im Wesentlichen 65 mm lang sind.

11. Positionierungs- und Stabilisierungsstruktur (3300) nach einem der Ansprüche 7 bis 10, wenn abhängig von Anspruch 6, wobei zwischen jeder Lasche und jedem lateralen Rand der jeweiligen Öffnung (3668) ein lateraler Abstand von weniger als 5 mm vorhanden ist.

12. Positionierungs- und Stabilisierungsstruktur (3300) nach einem der Ansprüche 1 bis 11, wobei die Textilhülle (3650) ein erstes Material (3670) auf einer den Patienten kontaktierenden Seite (3674, 3676) der Hülle (3363, 3650) und ein zweites Material (3672) auf einer den Patienten nicht kontaktierenden Seite (3674, 3676) der Hülle (3363, 3650) aufweist.

13. Positionierungs- und Stabilisierungsstruktur (3300) nach Anspruch 12, wobei das erste Material (3670) durch eine Naht (3678) mit dem zweiten Material (3672) verbunden ist, wobei die Naht (3678) dazu konfiguriert ist, Dehnung in der Umfangsrichtung zuzulassen.

14. Positionierungs- und Stabilisierungsstruktur (3300) nach einem der Ansprüche 1 bis 13, wobei der Rand (3351, 3669) der Öffnung (3668) mit Naht (3678)-Band versehen ist, um Ausfransen des Textils zu verringern oder zu verhindern.

15. Positionierungs- und Stabilisierungsstruktur (3300) nach einem der Ansprüche 1 bis 14, wobei die zwei Schläuche (3350) einstückig ausgebildet sind.

## Revendications

1. Structure de positionnement et de stabilisation (3300) servant à établir une force pour maintenir une structure de formation d'étanchéité (3100) dans une position thérapeutiquement efficace sur la tête d'un patient, la structure de formation d'étanchéité (3100) étant structurée et conçue pour former une étanchéité avec une région du visage du patient entourant une entrée des voies aériennes du patient pour une distribution étanche d'un flux d'air à une pression thérapeutique d'au moins 6 cmH₂O au-dessus de la pression d'air ambiant à au moins les narines du patient sur tout le cycle respiratoire du patient en utilisation, la structure de positionnement et de stabilisation (3300) comprenant :
deux tubes de distribution de gaz (3350) servant à recevoir le flux d'air d'un orifice de raccordement situé au sommet de la tête du patient et à distribuer le flux d'air à l'entrée des voies aériennes du patient par le biais de la structure de formation d'étanchéité (3100), les tubes de distribution de gaz (3350) étant structurés et conçus pour contacter, en utilisation, au moins une région de la tête du patient supérieure à un otobasion supérieur de la tête du patient,
la structure de positionnement et de stabilisation (3300) comprenant en outre un manchon textile allongé (3650) disposé autour des tubes de distribution de gaz (3350) et conçu pour être en contact, en utilisation, avec le visage du patient, le manchon (3650) comprenant une paroi (3666) dotée de deux ouvertures (3668) ménagées en son sein destinées à permettre au patient (1000) de visualiser une partie des tubes de distribution de gaz (3350) lorsque la structure de positionnement et de stabilisation (3300) n'est pas en utilisation,
dans laquelle la largeur de chaque ouverture (3668) est inférieure à 50 % de la circonférence des tubes de distribution de gaz (3350), lorsque l'ouverture (3668) et le tube (3350) sont mesurés à la même position longitudinale,
dans laquelle les tubes de distribution de gaz (3350) comprennent deux parties de coopération de sangle (3320) destinées à coopérer avec une sangle (3310), et le manchon textile (3650) est conçu de sorte que les parties de coopération de sangle (3320) fassent saillie à travers chacune des deux ouvertures respectives (3668).

2. Structure de positionnement et de stabilisation (3300) selon la revendication 1, dans laquelle le manchon textile (3650) est élastiquement souple dans une direction axiale et dans une direction circonférentielle, et a une souplesse dans la direction axiale plus grande que dans la direction circonférentielle.

3. Structure de positionnement et de stabilisation (3300) selon la revendication 1 ou la revendication 2, dans laquelle les ouvertures (3668) sont sensiblement ovales, elliptiques ou en forme de stade.

4. Structure de positionnement et de stabilisation (3300) selon l'une quelconque des revendications 1 à 3, dans laquelle les tubes de distribution de gaz (3350) comprennent une partie de concertina (3354) et une partie de non-concertina, dans laquelle la partie de non-concertina est située du côté opposé à la partie de concertina (3354) par rapport à l'orifice de raccordement (3360, 3600), dans laquelle le manchon textile (3650) est conçu de sorte que le bord complet de chacune des ouvertures (3668) repose sur la partie de non-concertina des tubes de distribution de gaz respectifs (3350).

5. Structure de positionnement et de stabilisation (3300) selon la revendication 4, dans laquelle le manchon (3363, 3650) s'étend jusqu'à l'extrémité (3358, 3808) de la partie de non-concertina du tube de distribution de gaz respectif (3350) qui est opposée à la partie de concertina (3354) .

6. Structure de positionnement et de stabilisation (3300) selon l'une quelconque des revendications 1 à 5, dans laquelle chacune des deux parties de coopération de sangle (3320) comprend une patte.

7. Structure de positionnement et de stabilisation (3300) selon l'une quelconque des revendications 1 à 6, dans laquelle un dégagement est présent entre le bord (3351, 3669) de chaque ouverture (3668) et la partie de coopération de sangle respective (3320).

8. Structure de positionnement et de stabilisation (3300) selon la revendication 7, dans laquelle le dégagement est dans la direction longitudinale.

9. Structure de positionnement et de stabilisation (3300) selon la revendication 8, dans laquelle le dégagement permet aux tubes de distribution de gaz (3350) de s'étirer, en utilisation, sans que la partie de coopération de sangle respective (3320) ne contacte le bord (3351, 3669) de l'ouverture respective (3668).

10. Structure de positionnement et de stabilisation (3300) selon l'une quelconque des revendications 1 à 9, dans laquelle chacune des deux ouvertures (3668) a une longueur de sensiblement 65 mm.

11. Structure de positionnement et de stabilisation (3300) selon l'une quelconque des revendications 7 à 10, lorsqu'elles dépendent de la revendication 6, dans laquelle un dégagement latéral inférieur à 5 mm est présent entre chaque patte et chaque bord latéral de l'ouverture respective (3668).

12. Structure de positionnement et de stabilisation (3300) selon l'une quelconque des revendications 1 à 11, dans laquelle le manchon textile (3650) comprend un premier matériau (3670) d'un côté en contact avec le patient (3674, 3676) du manchon (3363, 3650), et un second matériau (3672) d'un côté sans contact avec le patient (3674, 3676) du manchon (3363, 3650).

13. Structure de positionnement et de stabilisation (3300) selon la revendication 12, dans laquelle le premier matériau (3670) est relié au second matériau (3672) par une couture (3678), dans laquelle la couture (3678) est configurée pour permettre un étirement dans la direction circonférentielle.

14. Structure de positionnement et de stabilisation (3300) selon l'une quelconque des revendications 1 à 13, dans laquelle un galon (3678) est disposé sur le bord (3351, 3669) de l'ouverture (3668) pour réduire ou éliminer un effilochage du textile.

15. Structure de positionnement et de stabilisation (3300) selon l'une quelconque des revendications 1 à 14, dans laquelle les deux tubes (3350) sont formés d'une seule pièce.
